# EUROPEAN PATENT SPECIFICATION

(11) **EP 4 403 107 B1**
(45) Date of publication and mention of the grant of the patent: **07.10.2026**
(21) Application number: 23784739.7
(22) Date of filing: 03.04.2023
(51) Int. Cl.: A61B 5/1455, G01N 21/49

(54) **IN-BLOOD SUBSTANCE CONCENTRATION MEASUREMENT DEVICE AND IN-BLOOD SUBSTANCE CONCENTRATION MEASUREMENT METHOD**
VORRICHTUNG ZUR MESSUNG DER KONZENTRATION EINER BLUTSUBSTANZ UND VERFAHREN ZUR MESSUNG DER KONZENTRATION EINER BLUTSUBSTANZ
DISPOSITIF DE MESURE DE CONCENTRATION DE SUBSTANCE DANS LE SANG ET PROCÉDÉ DE MESURE DE CONCENTRATION DE SUBSTANCE DANS LE SANG

(30) Priority: 06.04.2022 JP 2022063273
(43) Date of publication of application: 24.07.2024
(73) Proprietor: Light Touch Technology Incorporated, Osaka-shi, Osaka, 540-0029 (JP)
(72) Inventor: YAMAKAWA, Koichi, Osaka-shi, Osaka 540-0029 (JP); OGAWA, Kanade, Osaka-shi, Osaka 540-0029 (JP); YAMAKAWA, Yoko, Osaka-shi, Osaka 540-0029 (JP)
(74) Representative: Grünecker Patent- und Rechtsanwälte PartG mbB
(86) International application number: PCT/JP2023/013841
(87) International publication number: WO 2023/195453

(56) References cited:
- WO-A1-2012/136982
- WO-A1-2016/117520
- JP-A- 2008 154 687
- JP-A- 2008 309 707
- JP-A- 2009 168 670
- JP-A- 2017 509 373
- US-A1- 2021 085 186

## Description

### [Technical Field]

The present disclosure relates to a device and a method for measuring, by means of a noninvasive measurement method, the concentration of substances that are included in blood flowing through blood vessels of a living body.

### [Background Art]

To prevent and treat lifestyle-related diseases, it is significant that the states of substances in blood, such as the blood sugar level and blood lipid level, be checked on a daily basis. In particular, for patients with diabetes, which is one of such lifestyle-related diseases, it is required that the concentration of glucose in blood be measured and the blood sugar level be managed on a daily basis in order to prevent complications, and an invasive method in which blood is sampled from a patient to chemically analyze the blood has been conventionally performed.

In contrast, in recent years, simple noninvasive methods have been proposed in which the state of blood in the body is optically analyzed without sampling blood (for example, Patent Literatures 1 and 2).

### [Citation List]

### [Patent Literature]

[Patent Literature 1] WO 2016/117520
[Patent Literature 2] JP 2009-168670A

Further substance-in-blood concentration measurement devices and substance-in-blood concentration measurement methods according to the state of the art are for instance described in JP2008-154687A and WO2012/136982A1.

### [Summary of Invention]

### [Technical Problem]

However, the substance-in-blood concentration measurement device disclosed in each of Patent Documents 1 and 2 has a structure for performing measurement by placing a measurement-target part into contact with a light-emission window provided on the outer surface of the measurement device. Thus, there is a problem that it is difficult to stably obtain sufficient measurement accuracy because measurement results may fluctuate as a result of a change in pressure applied to the measurement-target part, measurement position, etc., caused by a difference in the manner in which the measurement-target part is placed on the light-emission window.

The present disclosure has been made in view of the above-described problem, and an object thereof is to provide a substance-in-blood concentration measurement device and a substance-in-blood concentration measurement method with which a fluctuation in measurement results caused by a difference in the manner in which a measurement-target part is placed on the device can be suppressed and accurate measurement can be stably performed.

### [Solution to Problem]

According to the invention, there is provided a substance-in-blood concentration measurement device according to claim 1 and a substance-in-blood concentration measurement method according to claim 15, preferred embodiments being specified in dependent claims 2-14 and 16-22.

### [Advantageous Effects of Invention]

According to the substance-in-blood concentration measurement device and the substance-in-blood concentration measurement method pertaining to aspects of the present disclosure, a fluctuation in measurement results caused by a difference in the manner in which a measurement-target part is placed on the device can be suppressed and accurate measurement can be stably performed.

### [Brief Description of Drawings]

FIG. 1 is a schematic diagram illustrating a state of a substance-in-blood concentration measurement device 1 according to Embodiment 1 during measurement.
FIG. 2 is a side view illustrating a structure of the substance-in-blood concentration measurement device 1.
FIG. 3 is an enlarged view illustrating a portion of a living body placed on a base in FIG. 2.
FIG. 4 is a schematic diagram illustrating a structure of a light emission unit 20 in the substance-in-blood concentration measurement device 1.
FIG. 5 is a diagram for describing an overview of a light-receiving-side optical path in the substance-in-blood concentration measurement device 1.
FIG. 6 is a schematic diagram illustrating an overview of an optical path from the light emission unit 20 to a photodetector 30 in the substance-in-blood concentration measurement device 1.
FIG. 7 is a schematic diagram for describing an operation by the substance-in-blood concentration measurement device 1 for adjusting the length of an optical path from a laser-beam condensing region FA to the photodetector 30.
FIG. 8 is a diagram illustrating laser-beam emission positions on the surface of the living body in substance-in-blood-concentration measurement by the substance-in-blood concentration measurement device 1.
FIG. 9 is a flowchart illustrating one aspect of a substance-in-blood measurement operation by the substance-in-blood concentration measurement device 1.
FIG. 10 is a flowchart illustrating another aspect of the substance-in-blood measurement operation by the substance-in-blood concentration measurement device 1.
FIG. 11 is a flowchart illustrating yet another aspect of the substance-in-blood measurement operation by the substance-in-blood concentration measurement device 1.
FIG. 12 is a schematic diagram illustrating a state of a substance-in-blood concentration measurement device 1A according to Embodiment 2 during measurement.
FIG. 13 is a schematic diagram illustrating a structure of an example of the substance-in-blood concentration measurement device 1A.
FIG. 14 is a diagram illustrating results of a substance-in-blood concentration measurement test in which the example of the device 1A and an SMBG-based comparative example were used.
FIG. 15 is a schematic diagram illustrating a state of a substance-in-blood concentration measurement device 1B according to Embodiment 3 during measurement.

### [Description of Embodiments]

### <<Overview of Embodiments of Invention>>

A substance-in-blood concentration measurement device according to at least one embodiment of the present disclosure is
a substance-in-blood concentration measurement device that measures a concentration of a substance in blood included in the blood in a subject portion of a living body, the substance-in-blood concentration measurement device characterized by including:
a base having a main surface on which the living body can be placed;
a light emission unit that, from the main surface-side of the base, condenses and emits a laser beam to a specific region in the subject portion, the specific region being present in skin located on the reverse side of the living body from a skin surface on a side of the living body facing the main surface;
a photodetector that, on the main surface-side of the base, receives signal light that is reflected light based on the laser beam, and detects an intensity of the signal light, the signal light having lower light intensity than the laser beam in some wavelengths;
an imaging lens that is disposed in a position between the subject portion and the photodetector where a focused image of the signal light from a laser-beam condensing region in the subject portion can be formed on the photodetector; and
a measurement controller that measures a concentration of the substance in blood in the laser-beam condensing region based on the intensity of the signal light, wherein
a first angle formed by a normal to the surface of the skin of the subject portion and an optical path of the laser beam is different from a second angle formed by the normal and an optical path of the signal light from the laser-beam condensing region to the photodetector,
the position of the living body relative to the light emission unit is set so that the laser-beam condensing region and a blood-vessel region inwards of the epidermis in the subject portion overlap, and
the position of the photodetector relative to the living body is set so that an image of the signal light from the blood-vessel region overlapping the laser-beam condensing region is transferred by the imaging lens to form a focused image on a light receiving surface of the photodetector.

According to this structure, a fluctuation in measurement results caused by a difference in the manner in which a measurement-target part is placed on the device can be suppressed and accurate measurement can be stably performed by, from the main surface-side of the base, condensing and emitting a laser beam to a specific region in a subject portion that is present on the reverse side of a living body from a skin surface on a side of the living body facing the main surface, and, on the main surface-side of the base, receiving and measuring reflected light based on the laser beam, i.e., signal light having lower light intensity than the laser beam in some wavelengths.

Furthermore, according to another aspect, in any of the above-described aspects, the base is capable of adjusting the position of the living body relative to the light emission unit by changing the height of the main surface in a direction perpendicular to the main surface.

According to this structure, the positional relationship of the living body relative to the light emission unit can be adjusted in accordance with the shape of the individual living body so that the laser beam is condensed and emitted to the blood-vessel region inwards of the epidermis in the subject portion. Thus, the position of the living body relative to the light emission unit is regulated so that the laser-beam condensing region and the blood-vessel region inwards of the epidermis in the subject portion overlap in an XZ plane.

Furthermore, according to another aspect, in any of the above-described aspects, the position of the photodetector relative to the living body in a direction intersecting the optical path of the signal light is capable of being adjusted by changing the position of the photodetector in said direction.

According to this structure, the position of the photodetector can be adjusted so that the center of a screen of the photodetector and the position (laser-beam condensing region) in the subject portion to which the laser beam is condensed and emitted are substantially aligned or partially overlap in the direction perpendicular to the optical path to the photodetector.

Furthermore, according to another aspect, in any of the above-described aspects, the base is capable of adjusting the first angle and the second angle simultaneously by changing an angle of the main surface relative to the optical path of the laser beam within a plane that is defined by the optical path of the laser beam and the optical path from the subject portion to the photodetector.

According to this structure, an incident angle and an installation angle relative to the subject portion can be adjusted simultaneously in accordance with the shape of the individual living body.

Furthermore, according to another aspect, in any of the above-described aspects, the position of the photodetector relative to the living body in a direction along the optical path of the signal light is capable of being adjusted by changing the position of the photodetector in said direction, and the position of the photodetector is adjusted based on the concentration of the substance in blood so that the image of the signal light from the blood-vessel region is transferred by the imaging lens to form a focused image on the light receiving surface of the photodetector.

According to this structure, the position of the photodetector in the direction parallel to the optical path can be adjusted so that an image at a depth corresponding to the blood-vessel region in the subject portion is transferred as an image of equivalent size on the screen of the photodetector.

Due to this, the photodetector can receive signal light having sufficiently high intensity relative to background light as a result of a focused image of the signal light from the laser-beam condensing region being formed on the screen of the photodetector by the imaging lens. Thus, a high S/N ratio is realized and accurate measurement can be performed.

Furthermore, according to another aspect, in any of the above-described aspects, the light emission unit is capable of selectively emitting: a first laser beam for a target measurement that is absorbed by a first substance in blood that is a measurement-target substance; and a second laser beam for a reference measurement that is absorbed by a second substance in blood that is a reference substance, and an absorption rate at which the second laser beam is absorbed by the second substance in blood in the reference measurement is higher than an absorption rate at which the first laser beam is absorbed by the first substance in blood in the target measurement.

According to this structure, accurate measurement of the concentration of the first substance in blood can be performed stably at all times regardless of individual differences between subject persons in the depth-direction position of blood vessels from the surface of the skin, by condensing and emitting the first laser beam from the light emission unit and receiving signal light from the laser-beam condensing region by means of the photodetector at a photodetector position where signal light from a blood vessel was detected. Thus, accurate measurement can be performed stably regardless of individual differences in the measurement target.

Furthermore, according to another aspect, in any of the above-described aspects, a concentration of the reference substance in blood is more stable than a concentration of the measurement-target substance in blood.

According to this structure, the accuracy of the measurement of the measurement target can be improved by performing the target measurement after performing the reference measurement for the reference substance.

Furthermore, according to another aspect, in any of the above-described aspects, the measurement controller is capable of measuring, based on emission of the second laser beam, a concentration of the second substance in blood in a state in which the specific region is included in the blood-vessel region in the subject portion, and
is capable of measuring, based on emission of the first laser beam, a concentration of the first substance in blood in the specific region as a concentration of the first substance in blood in the measurement-target portion.

According to this structure, the concentration of the first substance in blood can be measured in a state in which the laser-beam condensing region is included in a blood-vessel region in the subject portion.

Furthermore, according to another aspect, in any of the above-described aspects, a condenser lens that is positioned between the light emission unit and the subject portion in the optical path of the laser beam, and that condenses the laser beam to the laser-beam condensing region is included.

According to this structure, a fluctuation in substance-in-blood-concentration measurement results between instances of measurement can be reduced.

Furthermore, according to another aspect, in any of the above-described aspects, within a section from the base to the photodetector in the optical path from the subject portion to the photodetector, the signal light propagates through a space except within a section in which the signal light passes through the imaging lens, and
within a section from the light emission unit to the base in the optical path from the light emission unit to the subject portion, the laser beam propagates through a space except within a section in which the laser beam passes through the condenser lens.

According to this structure, compared to the conventional device disclosed in Patent Literature 1, in which a waveguide is used, a false-signal (noise) component produced by reflected light being scattered at the surface of the skin can be reduced, and the S/N ratio in optical measurement can be improved.

Furthermore, according to another aspect, in any of the above-described aspects, an image-capturing means that captures an image including the living body is further included, wherein
the measurement controller detects an image portion corresponding to the living body from the acquired image and calculates a positional deviation amount from a reference position where the image portion should be located, and
the base is capable of changing the height of the main surface in a perpendicular direction so as to compensate for the positional deviation amount.

According to this structure, the position of the living body relative to the light emission unit can be easily adjusted in accordance with the shape of the individual living body.

Furthermore, according to another aspect, in any of the above-described aspects, an image-capturing means that captures an image including the living body is further included, wherein
the measurement controller detects an image portion corresponding to the living body from the acquired image and calculates an angular deviation amount from a reference angle in which the image portion should be oriented, and
the base is capable of changing the angle of the main surface relative to the optical path of the laser beam so as to compensate for the angular deviation amount.

According to this structure, the incident angle and the installation angle relative to the subject portion can be adjusted with ease simultaneously in accordance with the shape of the individual living body.

Furthermore, according to another aspect, in any of the above-described aspects, a light-emission-angle adjustment mechanism that holds the light emission unit and the photodetector and that, by rotating, changes the angle of the optical path of the signal light and the angle of the optical path of the laser beam with respect to the surface of the skin of the living body within a laser-beam incident plane is further included.

According to this structure, the incident angle of the laser beam emitted from the light emission unit on the subject portion and the installation angle of the photodetector relative to the subject portion can be adjusted simultaneously, and the positions and angles of the photodetector and the light emission unit can be regulated so that the optical path of the laser beam and the optical path of the signal light intersect at a predetermined position.

Furthermore, according to another aspect, in any of the above-described aspects, a position adjustment mechanism that changes the position of the light-emission-angle adjustment mechanism within the laser-beam incident plane is further included.

According to this structure, the point of intersection between the optical path of the signal light and the optical path of the laser beam relative to the surface of the skin of the living body can be moved to a position that is to become the subject portion of the living body. Thus, a subject person can perform substance-in-blood concentration measurement more easily without being constrained by the manner in which the measurement-target part is to be placed on the device, the laser incident angle, or the photodetector installation angle.

Furthermore, a substance-in-blood concentration measurement method according to at least one embodiment of the present disclosure is a substance-in-blood concentration measurement method for measuring a concentration of a substance in blood included in the blood in a subject portion of a living body, the substance-in-blood concentration measurement method including:
placing the living body on a main surface of a base, and, from the main surface-side of the base, condensing and emitting a laser beam for a target measurement to a specific region in the subject portion by means of a light emission unit, the specific region being present in skin located on the reverse side of the living body from a skin surface on a side of the living body facing the main surface, and the laser beam for the target measurement being absorbed by a substance in blood that is a measurement-target substance;
on the main surface-side of the base, using an imaging lens that is positioned between the subject portion and a photodetector to form a focused image of signal light on a photodetector, the signal light being reflected light of the laser beam, being emitted from a laser-beam condensing region in the subject portion, and having lower light intensity than the laser beam in some wavelengths; and
receiving the signal light by means of the photodetector and measuring a concentration of the substance in blood in the specific region based on the intensity of the received signal light, wherein
a first angle formed by a normal to the surface of the skin of the subject portion and an optical path of the laser beam is different from a second angle formed by the normal and an optical path of the signal light from the specific region to the photodetector,
the position of the living body relative to the light emission unit is set so that the laser-beam condensing region and a blood-vessel region inwards of the epidermis in the subject portion overlap, and
the position of the photodetector relative to the living body is set so that an image of the signal light from the blood-vessel region overlapping the laser-beam condensing region is transferred by the imaging lens to form a focused image on a light receiving surface of the photodetector.

According to this structure, a substance-in-blood concentration measurement method can be provided with which a fluctuation in measurement results caused by a difference in the manner in which a measurement-target part is placed on a device can be suppressed and accurate measurement can be stably performed.

Furthermore, according to another aspect, in any of the above-described aspects, prior to the measuring, the position of the living body relative to the light emission unit is adjusted by changing the height of the main surface of the base in a direction perpendicular to the main surface.

Furthermore, according to another aspect, in any of the above-described aspects, prior to the measuring, the position of the photodetector relative to the living body in a direction intersecting the optical path of the signal light is adjusted by changing the position of the photodetector in said direction.

Furthermore, according to another aspect, in any of the above-described aspects, prior to the target measurement, the first angle and the second angle are adjusted simultaneously based on the concentration of the substance in blood by performing a reference measurement in which the concentration of the substance in blood in the specific region is measured while changing the angle of the main surface within a plane that is defined by the optical path of the laser beam and the optical path from the subject portion to the photodetector.

Furthermore, according to another aspect, in any of the above-described aspects, prior to the target measurement, the position of the photodetector relative to the living body is adjusted in a direction along the optical path of the signal light by performing a reference measurement in which the concentration of the substance in blood is measured while changing the position of the photodetector along the optical path of the signal light, wherein
in the adjusting, the position of the photodetector is adjusted based on the concentration of the substance in blood so that an image of the signal light from the blood-vessel region is transferred by the imaging lens to form a focused image on the light receiving surface of the photodetector.

Furthermore, according to another aspect, in any of the above-described aspects, when the laser beam, the substance in blood, and the concentration of the substance in blood are respectively referred to as a first laser beam, a first substance in blood, and a concentration of the first substance in blood,
in the reference measurement:
a second laser beam for the reference measurement that is absorbed by a second substance in blood that is a reference substance is emitted from the light emission unit to the irradiated region;
using the imaging lens, a focused image is formed on the photodetector from signal light of the second laser beam reflected from the specific region;
the signal light of the second laser beam is received by means of the photodetector, and a concentration of the second substance in blood based on the signal light is measured as a concentration of the second substance in blood in the measurement-target portion; and
an absorption rate at which the second laser beam is absorbed by the second substance in blood is higher than an absorption rate at which the first laser beam is absorbed by the first substance in blood in the target measurement.

Furthermore, a program according to at least one embodiment of the present disclosure is
a program that causes a computer to execute substance-in-blood concentration measurement processing of measuring a concentration of a substance in blood included in the blood in a subject portion of a living body, wherein
the substance-in-blood concentration measurement processing being characterized by including:
   placing the living body on a main surface of a base, and, from the main surface-side of the base, condensing and emitting a laser beam for a target measurement to a specific region in the subject portion by means of a light emission unit, the specific region being present in skin located on the reverse side of the living body from a skin surface on a side of the living body facing the main surface, and the laser beam for the target measurement being absorbed by a substance in blood that is a measurement-target substance;
   on the main surface-side of the base, using an imaging lens that is positioned between the subject portion and a photodetector to form a focused image of signal light on a photodetector, the signal light being reflected light of the laser beam, being emitted from a laser-beam condensing region in the subject portion, and having lower light intensity than the laser beam in some wavelengths; and
   receiving the signal light by means of the photodetector and measuring a concentration of the substance in blood in the specific region based on the intensity of the received signal light, wherein
   a first angle formed by a normal to the surface of the skin of the subject portion and an optical path of the laser beam is different from a second angle formed by the normal and an optical path of the signal light from the specific region to the photodetector,
   the position of the living body relative to the light emission unit is set so that the laser-beam condensing region and a blood-vessel region inwards of the epidermis in the subject portion overlap, and
   the position of the photodetector relative to the living body is set so that an image of the signal light from the blood-vessel region overlapping the laser-beam condensing region is transferred by the imaging lens to form a focused image on a light receiving surface of the photodetector.

According to this structure, a program can be provided with which a fluctuation in measurement results caused by a difference in the manner in which a measurement-target part is placed on a device can be suppressed and accurate measurement can be stably performed.

### <<Embodiment 1>>

A substance-in-blood concentration measurement device 1 according to the present embodiment will be described with reference to the drawings. Here, in this description, the positive and negative directions in the height direction may be respectively referred to as the "upward" and "downward" directions, and a surface facing the positive direction in the height direction and a surface facing the negative direction in the height direction may be respectively referred to as a "front" surface and a "rear" surface. Furthermore, members in the drawings are not necessarily drawn to scale. Furthermore, "perpendicular" and "parallel" may indicate angles with differences from the angles 90° and 0°, respectively, as long as functions are not substantially impaired thereby. Furthermore, in this description, numerical ranges indicated using the symbol "-" include the values at both ends of the numerical ranges. Further, only preferred examples of materials, numerical values, etc., are indicated in the present embodiment, and there is no limitation thereto.

### <Overall Structure>

The substance-in-blood concentration measurement device 1 (also referred to hereinafter as "device 1") is a medical device that noninvasively measures the concentration of a substance in the blood of a living body in a blood-vessel region by emitting a laser beam having a specific wavelength from a light source to the blood-vessel region of the living body and detecting the intensity of signal light from the blood-vessel region. As the laser beam, light of a specific wavelength capable of being absorbed by the substance to be measured is used. When the concentration of the substance in blood is high, signal light having lower light intensity than the laser beam in some wavelengths is emitted from the blood-vessel region due to absorption by the substance. Thus, the device 1 measures the concentration of the substance in blood by measuring the intensity of the signal light using a photodetector.

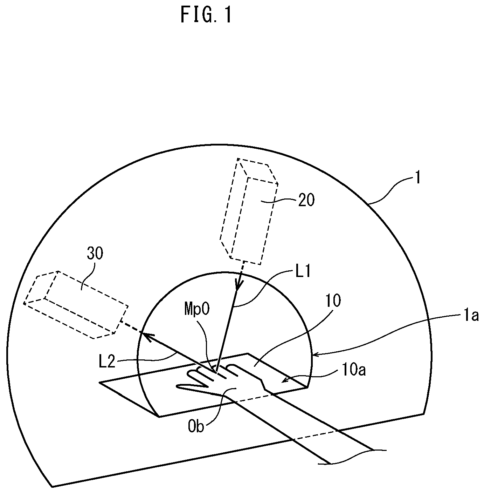

FIG. 1 is a schematic diagram illustrating a state of the device 1 during measurement. The device 1 is a substance-in-blood concentration measurement device in which, when a subject person inserts a living body Ob (for example, a finger) that is the subject into an opening 1a provided in the front surface of the device 1 and places the living body Ob on a main surface 10a of a base 10 inside the device 1, a laser beam L1 is emitted from a light emission unit 20 that is disposed on the main surface 10a-side of the base 10 to a subject portion Mp0 present in the skin located on the reverse side of the living body Ob from a skin surface on a side of the living body Ob facing the main surface 10a, and signal light L2 obtained by the laser beam L1 being reflected by the subject portion Mp0 is received and measured using a photodetector 30 that is disposed on the main surface 10a-side of the base 10.

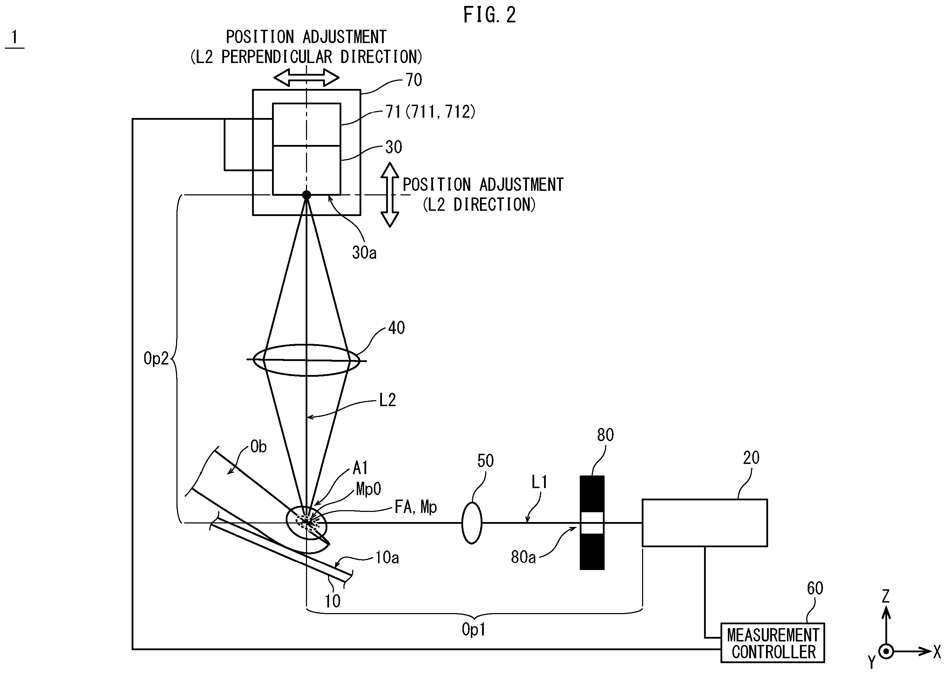

FIG. 2 is a schematic diagram illustrating a structure of the device 1. As illustrated in FIG. 2, the device 1 includes the base 10, the light emission unit 20, the photodetector 30, a condenser lens 50, an imaging lens 40, a measurement controller 60, a light detection unit 70, and a diaphragm 80.

Structures of the elements of the device 1 will be described below.

### <Structures of Elements>

### (Base 10)

The base 10 is a plate-shaped member including the main surface 10a, which is the upward-facing surface of the base 10. During measurement, the living body Ob is placed on the main surface 10a. The base 10 is a guide member that stabilizes the position of the subject portion Mp0 of the living body Ob during measurement, and also regulates a blood-vessel region Mp that is included in the subject portion Mp0 of the living body Ob to a predetermined position and angle that are suitable for irradiation with the laser beam L1.

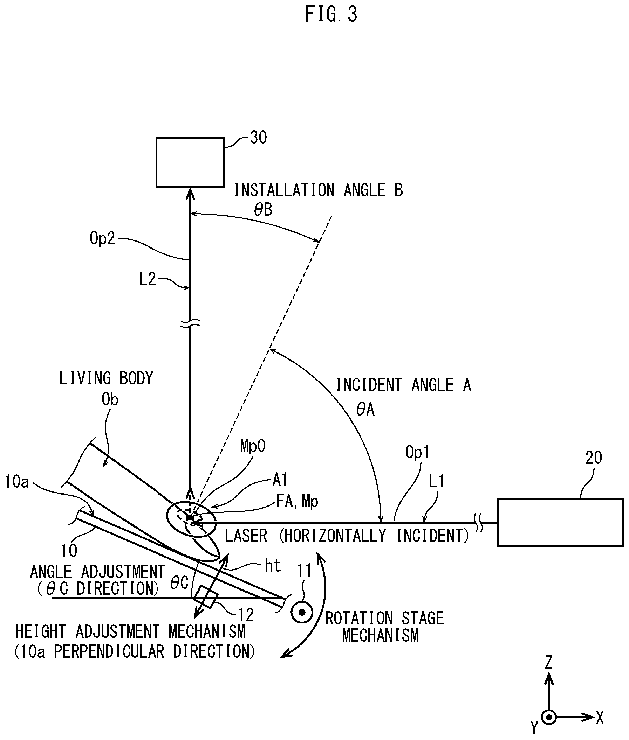

FIG. 3 is an enlarged view illustrating the portion of the living body placed on the base in FIG. 2.

In the present embodiment, the finger serving as the living body Ob is placed on the main surface 10a in a state in which the palm side of the finger is placed into contact with the main surface 10a, and measurement is performed in a state in which the back side of the finger is the subject portion Mp0. That is, when seen from the skin surface on the side of the living body Ob facing the main surface 10a, the subject portion Mp0 is present in the skin located on the reverse side (A1 in FIGS. 2 and 3) of the living body Ob from the skin surface.

A mark indicating a measurement position is provided on the main surface 10a of the base 10. By placing the palm side of the finger serving as the living body Ob into contact with the main surface 10a in a state in which the living body Ob is positioned with respect to the mark, the subject portion Mp0 present in the skin on the back side of the finger serving as the living body Ob can be held in a state in which the subject portion Mp0 is separated from the main surface 10a of the base 10 by a predetermined distance. Furthermore, the position of the mark on the main surface 10a is defined so that, in a state in which the angle of the main surface 10a is regulated to a predetermined angle, the subject portion Mp0 of the living body Ob positioned with respect to the mark is in a predetermined position relative to the light emission unit 20 and the photodetector 30.

Furthermore, the base 10 is disposed so that the laser beam L1 emitted from the light emission unit 20 is incident from the main surface 10a-side. Furthermore, the relative angle of the main surface 10a relative to the light emission unit 20 is regulated so that an incident angle A of the laser beam L1 on the subject portion Mp0 of the living body Ob equals a predetermined angle θA.

Here, the incident angle A indicates an angle θA, with respect to the normal to the surface of the skin of the living body Ob placed on the main surface 10a of the base 10, of an optical path Op1 of the laser beam L1 from the light emission unit 20 to a region (also referred to hereinafter as "laser-beam condensing region FA") in the subject portion Mp0 to which the laser beam L1 is condensed.

The relative angle of the main surface 10a of the base 10 relative to the photodetector 30 is simultaneously regulated so that an installation angle B of the photodetector 30 relative to the subject portion Mp0 of the living body Ob equals a predetermined angle θB.

Here, the installation angle B indicates an angle, with respect to the normal to the surface of the skin of the living body Ob placed on the main surface 10a of the base 10, of an optical path Op2 of the signal light L2 from the laser-beam condensing region FA in the subject portion Mp0 to the photodetector 30. Here, the angle θA differs from the installation angle B.

That is, in the device 1, the position of the light emission unit 20 relative to the main surface 10a of the base 10 is regulated, and, simultaneously, the position of the photodetector 30 relative to the main surface 10a of the base 10 and the light emission unit 20 is regulated via a later-described movable mechanism 71. Thus, in the device 1, the installation angle B can be set to the predetermined angle θB in a state in which the incident angle A is equal to the predetermined angle θA.

By using the base 10 having this structure, the incident angle A of the laser beam L1 relative to the subject portion Mp0, the installation angle B of the photodetector 30 relative to the subject portion Mp0, and the position in the subject portion Mp0 to which the laser beam L1 is emitted can be approximately regulated to near the appropriate values thereof that are necessary for measurement by simply placing the palm side of the finger serving as the living body Ob into contact with the main surface 10a.

Furthermore, the base 10 includes a rotation stage mechanism 11 that, within a plane (also referred to hereinafter as "laser-beam incident plane") that is determined by the optical path of the laser beam L1 emitted from the light emission unit 20 and the optical path of the signal light L2 from the subject portion to the photodetector 30, changes an angle θC of the main surface 10a relative to the optical paths of the laser beam L1 and the signal light L2.

The rotation stage mechanism 11 includes an angle changing mechanism with an internal motor. The motor is driven based on a control signal from the measurement controller 60, and the rotation stage mechanism 11 thereby actuates the angle changing mechanism to change the angle θC of the main surface 10a, and outputs information regarding the angle θC to the measurement controller 60.

By means of this rotation stage mechanism 11, the incident angle A and the installation angle B relative to the subject portion Mp0 can be adjusted simultaneously in accordance with the shape or size of the individual living body Ob even if the shape of the surface of the skin of the living body Ob differs depending on the subject person.

Here, the positional relationship between the main surface 10a, the center of rotation of the rotation stage mechanism 11, and the optical path of the laser beam L1 may be defined so that, in a state in which the living body Ob is positioned with respect to the mark on the main surface 10a, the subject portion Mp0 present in the skin on the back side of the finger moves substantially parallel to the direction of the normal to the skin surface on the back side of the finger as the angle θC changes due to rotation.

Thus, the position in the living body Ob to which the laser beam L1 is emitted can be suppressed from changing when the incident angle A and the installation angle B relative to the subject portion Mp0 are adjusted by changing the angle θC.

Furthermore, the base 10 includes a height adjustment mechanism 12 so that the base 10 can change a height ht in the direction perpendicular to the main surface 10a. For example, as the height adjustment mechanism 12, a known linear motion mechanism such as a lead screw or a linear motor can be used. The height adjustment mechanism 12 is driven based on a control signal by the measurement controller 60. The height adjustment mechanism 12 actuates the linear motion mechanism to change the height ht of the base 10, and outputs information regarding the height ht to the measurement controller 60.

By means of this height adjustment mechanism 12, the positional relationship of the subject portion Mp0 in the living body Ob relative to the light emission unit 20 can be adjusted in accordance with the shape or size of the individual living body Ob so that the laser beam is condensed and emitted to the blood-vessel region Mp located inward of the epidermis in the subject portion Mp0. Thus, the position of the living body Ob relative to the light emission unit 20 is regulated so that the laser-beam condensing region FA and the blood-vessel region Mp located inward of the epidermis in the subject portion Mp0 overlap within the XZ plane.

Furthermore, by adjusting the height ht using the height adjustment mechanism 12, a change in the distance from the main surface 10a to the subject portion Mp0 (present in the skin on the back side of the finger serving as the living body Ob) due to the thickness of the finger serving as the living body Ob can be absorbed. Thus, a difference in the thickness of the finger serving as the living body Ob, which differs depending on the subject person, can be absorbed.

Due to this, even if the subject person's finger serving as the living body Ob is thick (or thin), the position in the subject portion Mp0 of the living body Ob to which the laser beam L1 is emitted, the incident angle A of the laser beam L1 relative to the subject portion Mp0, and the installation angle B of the photodetector 30 relative to the subject portion Mp0 can be regulated to near the appropriate values thereof necessary for measurement by adjusting the height ht, by means of the height adjustment mechanism 12, in the direction in which the excess or deficiency in the thickness of the finger is compensated for.

### (Light Emission Unit 20)

The light emission unit 20 is a light source that emits a laser beam L1 having a specific wavelength to the living body, i.e., the subject portion Mp0 of the living body Ob. The light emission unit 20 is capable of emitting a laser beam for target measurement (also referred to hereinafter as "first laser beam") that oscillates at a wavelength that is absorbed by a first substance in blood that is the measurement-target substance (also referred to hereinafter as "first substance in blood").

Furthermore, in the device 1, the light emission unit 20 is capable of modulating laser-beam wavelength to selectively emit the laser beam for target measurement and a laser beam for reference measurement (also referred to hereinafter as "second laser beam") that oscillates at a wavelength that is absorbed by a second substance in blood that is a reference substance (also referred to hereinafter as "second substance in blood"). Thus, the type of substance in blood that can be detected is changed.

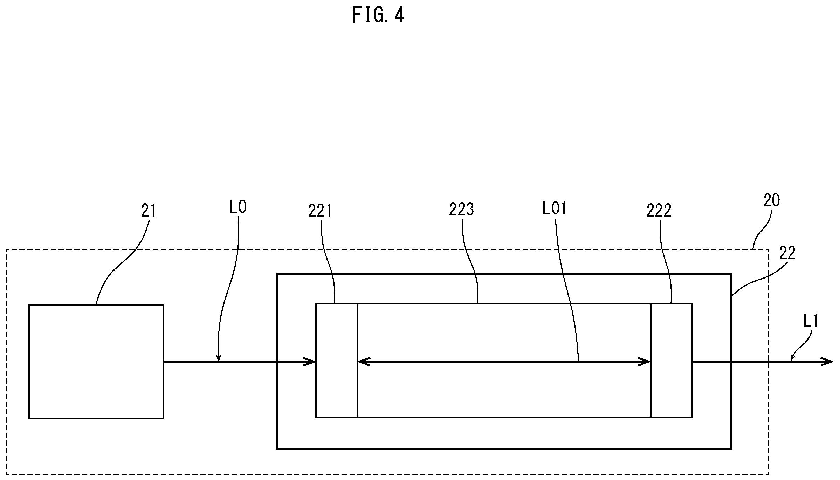

FIG. 4 is a schematic diagram illustrating a structure of the light emission unit 20 in the substance-in-blood concentration measurement device 1. As illustrated in FIG. 4, the light emission unit 20 includes a light source 21 that oscillates pulsed pump light L0 having a shorter wavelength than mid-infrared light, and an optical parametric oscillator (OPO) 22 that converts the wavelength into a longer wavelength and amplifies the pump light L0 to emit the resultant light as the laser beam L1. In the OPO 22, as a result of the pump light L0 being input to an internal nonlinear optical crystal, light of two different wavelengths is oscillated, resulting in signal light having a shorter wavelength and idler light having a longer wavelength being generated.

The light emission unit 20 outputs the idler light out of the two types of light as the laser beam L1 to a later stage where the laser beam L1 is used to measure the blood sugar level. A structure disclosed in a known document, e.g., JP 2010-281891, may be used for the OPO 22.

In the present embodiment, as one example, glucose may be adopted, for example, as the first substance in blood that is the measurement-target substance. In such a case, light having a wavelength selected from mid-infrared light is emitted as the laser beam. That is, as the wavelength oscillated by optical parametric oscillation, mid-infrared light is used due to being a wavelength that is absorbed to a greater extent by glucose than conventionally used near-infrared light, and a predetermined wavelength selected from the range of 2.5-12 µm, inclusive, may be adopted. A predetermined wavelength selected from the range of 6.0-12 µm, inclusive, is more preferable.

Specifically, in the present embodiment, the selected wavelength is 9.26 µm. For example, a wavelength within the range of 9.26 ± 0.05 µm (9.21-9.31 µm, inclusive) may be adopted. Alternatively, the wavelength may be selected from within the range of -0.05 µm to +0.05 µm, inclusive, from 7.05 µm, 7.42 µm, 8.31 µm, 8.7 µm, 9.0 µm, 9.57 µm, 9.77 µm, 10.04 µm, or 10.92 µm.

Thus, the glucose concentration in the blood of the subject can be measured as the blood sugar level. In this case, it is necessary to measure the glucose concentration in blood vessels in the skin, and mid-infrared light, which does not readily penetrate deeply into the body, is emitted directly to blood vessels (capillary vessels) in the skin.

Mid-infrared light has lower transmittance into the body than near-infrared light conventionally used to measure the blood sugar level. Thus, by identifying the position of blood vessels in the skin and emitting mid-infrared light thereto, an effect can be obtained that observation of only the blood-vessel portion is possible, in which case the observation is less susceptible to the influence of other body components present deeper in the body. Furthermore, by using mid-infrared light, an effect can also be obtained that the negative influence of overlaps of combination tones and overtones of fundamental vibrations on the measurement is reduced, and glucose can be measured more accurately than with near-infrared light.

On the other hand, as the reference substance (second substance in blood) measured in the reference measurement, a substance in blood satisfying the following is selected. That is, the substance has a higher laser-beam absorption rate than the measurement-target substance and thus has high measurement sensitivity, and the concentration of the substance in blood is very stable, resulting in less fluctuation in measurement results.

That is, the absorption rate at which the laser beam in the reference measurement is absorbed by the reference substance (second substance in blood) is preferably higher than the absorption rate at which the laser beam for target measurement is absorbed by the measurement-target substance (first substance in blood) in the target measurement. Additionally/alternatively, the concentration of the reference substance (second substance in blood) in blood is preferably more stable than the concentration of the measurement-target substance (first substance in blood) in blood.

The accuracy of the measurement of the measurement target can be improved by performing the target measurement after performing the reference measurement for the reference substance having this structure.

In the present embodiment, hemoglobin can be selected as one example of the reference substance (second substance in blood). By detecting hemoglobin in blood vessels, the positions of capillary vessels in the living body can be detected, and the most-suitable position of the measurement-target portion in the subject portion Mp0 of the living body Ob for performing the measurement of the measurement-target substance (first substance in blood) can be specified.

When hemoglobin is adopted as the first substance in blood or the second substance in blood, the laser beam to be emitted for measurement is light having a wavelength selected from mid-infrared light, and the wavelength is a predetermined wavelength selected from the range of 5.0-12 µm, inclusive.

Specifically, for example, a wavelength within the range of 8.00 ± 0.1 µm (7.9-8.1 µm, inclusive) may be adopted. Alternatively, the wavelength may be selected from: the range of 5.26-6.76 µm, inclusive; within the range of -0.1 µm to +0.1 µm, inclusive, from 7.17 µm; within the range of -0.1 µm to +0.1 µm, inclusive, from 7.58 µm; the range of 7.58-8.33 µm, inclusive; or within the range of - 0.1 µm to +0.1 µm, inclusive, from 8.55 µm.

The light source 21 may include a Q-switched Nd:YAG laser (oscillation wavelength: 1.064 µm) or a Q-switched Yb:YAG laser (oscillation wavelength: 1.030 µm). Thus, the light source 21 can oscillate pulsed pump light L0 having a shorter wavelength than mid-infrared light. The pump light L0 may have, for example, a pulse width of approximately 8 ns and a frequency of 10 Hz or higher.

Furthermore, because a Q-switched Nd:YAG laser or Yb:YAG laser operates as a passive Q-switch that switches passively using a saturable absorber, the light source 21 can be simplified and reduced in size.

As illustrated in FIG. 4, the OPO 22 includes an incident-side semi-transmissive mirror 221, an output-side semi-transmissive mirror 222, and a nonlinear optical crystal 223, and the nonlinear optical crystal 223 is disposed inside an optical resonator formed by disposing the incident-side semi-transmissive mirror 221 and the output-side semi-transmissive mirror 222 so as to face one another. Light L01 passing through the incident-side semi-transmissive mirror 221 enters the nonlinear optical crystal 223 and is converted into light having a wavelength determined by the nonlinear optical crystal 223, and is also optically parametrically amplified between the incident-side semi-transmissive mirror 221 and the output-side semi-transmissive mirror 222. The amplified light passes through the output-side semi-transmissive mirror 222 and is output as the laser beam L1.

In the nonlinear optical crystal 223, AgGaS suitable for wavelength conversion is used under phase-matching conditions. The wavelength of the oscillated laser beam L1 can be adjusted by adjusting the type and matching conditions of the nonlinear optical crystal 223. GaSe, ZnGeP₂, CdSiP₂, LiInS₂, LiGaSe₂, LiInSe₂, LiGaTe₂, etc., may be used as the nonlinear optical crystal. The laser beam L1 emitted from the OPO 22 is provided with a repetition frequency corresponding to the pump light L0, e.g., a pulse width of approximately 8 ns, and a high-intensity peak output of 10 W to 1 kW, inclusive, can be realized by the short pulse width.

As a result of the light source 21 and the OPO 22 being used in the light emission unit 20 as described above, the laser beam L1, which has a high intensity that is approximately 10³ to 10⁵ times the intensity of a conventional light source such as a quantum cascade laser, for example, can be obtained.

A device in which the wavelength of light emitted by the light emission unit 20 is changed can be realized by either: changing the configuration of the oscillation wavelength of the OPO 22 in the light emission unit 20 and changing the phase matching conditions of the nonlinear crystal 223 in the OPO 22; or changing the OPO 22 to that in which the phase matching conditions of the nonlinear crystal 223 are different.

According to this structure, the blood sugar level can be measured using mid-infrared light having low transmittance into the body.

The light emission unit 20 is electrically connected to the later-described measurement controller 60, and outputs the laser beam L1 based on a control signal from the measurement controller 60.

### (Condenser Lens 50)

As illustrated in FIG. 2, the condenser lens 50 (also referred to hereinafter as "second lens") for condensing emitted light to a specific region in the subject portion Mp0 is disposed on the optical path Op1 of the laser beam L1 from the light emission unit 20 to the subject portion Mp0 of the living body Ob. Within a section of the optical path Op1 from the light emission unit 20 to the surface of the living body Ob, the laser beam L1 propagates through a space filled with a gas, etc., for example, except within a section in which the laser beam L1 passes through the condenser lens 50.

In the device 1, the condenser lens 50 is optically designed so that, in a state in which the palm side of the finger serving as the living body Ob is placed on the main surface 10a so as to be positioned with respect to the predetermined position on the main surface 10a, the laser beam L1 emitted from the light emission unit 20 located on the main surface 10a-side of the base 10 is condensed and emitted to a specific region in the subject portion Mp0 located on the reverse side of the living body from the skin surface facing the main surface 10a.

Furthermore, the condenser lens 50 is optically designed so that the region to which the laser beam L1 is condensed is set to a depth corresponding to the blood-vessel region Mp located inward of the epidermis in the subject portion Mp0, or that is a portion of the living body located inward of the epidermis, such as the dermis for example. In the present description, the region in the subject portion Mp0 to which the laser beam L1 is condensed is referred to as the laser-beam condensing region FA as already mentioned above.

The incident angle A of the laser beam L1 on the subject portion Mp0 is determined by the angle of the optical path Op1 of the laser beam L1 from the light emission unit 20 to the laser-beam condensing region FA with respect to the normal to the portion of the surface of the skin of the living body Ob placed on the main surface 10a of the base 10 on which the laser beam L1 is incident.

In the present embodiment, the incident angle θA may be set to 45 degrees or more, and may further be set within the range of 60-70 degrees, inclusive, for example.

Here, a beam splitter (unillustrated) formed from a semi-transmissive mirror may be disposed between the light emission unit 20 and the condenser lens 50 to split a portion of the laser beam L1 as a reference signal, and the change in intensity of the laser beam L1 may be detected using a photodetector for monitoring (unillustrated) and be used in detection-signal normalization processing by the photodetector 30. This enables the output of the photodetector 30 to be compensated for based on the change in intensity of the laser beam L1.

The laser beam L1 having passed through the condenser lens 50 enters the living body Ob on the main surface 10a of the base 10 and passes through the epithelial interstitial tissue of the living body to be scattered or diffusely reflected. The signal light L2 so produced passes through the base 10 once again and is emitted toward the photodetector 30.

### (Diaphragm 80)

The diaphragm 80 is disposed in a section between the light emission unit 20 and the condenser lens 50 of the optical path Op1 of the laser beam L1 from the light emission unit 20 to the subject portion Mp0 of the living body Ob. The diaphragm 80 is formed from a light-blocking plate-shaped member, and an opening 80a (aperture) is opened in the center portion thereof.

The center of the opening 80a is aligned with the optical axis of the laser beam L1. Furthermore, the beam diameter of the laser beam L1 may be restricted to approximately one third by the opening 80a, for example.

Furthermore, the diaphragm 80 may be disposed at a position where the distance between the condenser lens 50 and the light emission unit 20 is internally divided to a_{IN}:b_{IN}, where 1/f_{IN} = 1/a_{IN} + 1/b_{IN} holds true, and f_{IN} is the focal length of the condenser lens 50.

By providing the diaphragm 80 between the light emission unit 20 and the condenser lens 50 in the optical path Op1 in such a manner, the fluctuation in the emission position of the laser beam L1 emitted to the living body Ob can be reduced.

### (Imaging Lens 40)

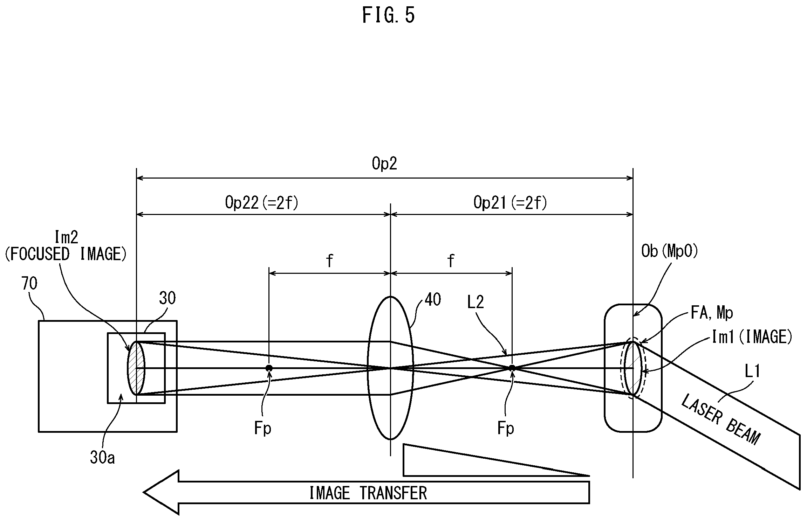

FIG. 5 is a diagram for describing an overview of a light-receiving-side optical path in the device 1, and is a schematic diagram illustrating the subject portion Mp0 of the living body Ob and a screen 30a of the photodetector 30 as seen in a cross-sectional view. As illustrated in FIGS. 2 and 5, the imaging lens 40 (also referred to hereinafter as "first lens") is disposed on the optical path Op2 of the signal light L2 from the laser-beam condensing region FA in the subject portion Mp0 of the living body Ob to the photodetector 30. The imaging lens 40 causes the signal light L2 emitted from the laser-beam condensing region FA as a result of diffuse reflection in the laser-beam condensing region FA in the subject portion Mp0 to form a focused image on the photodetector 30.

Within a section of the optical path Op2 from the surface of the living body Ob including the subject portion Mp0 to the photodetector 30, the signal light L2 propagates through a space, except within a section in which the signal light L2 passes through the imaging lens 40.

The imaging lens 40 is optically designed such that an image Im1 of the signal light L2 emitted as a result of diffuse reflection from a region in the subject portion Mp0 where the laser-beam condensing region FA and the blood-vessel region Mp have overlapped is transferred by the imaging lens 40 to form a focused image Im2 on the screen 30a of the photodetector 30.

In the present embodiment, a distance Op21 between the center of the imaging lens 40 and the laser-beam condensing region FA of the living body Ob and a distance Op22 between the screen 30a of the photodetector 30 and the center of the imaging lens 40 are equivalent.

Thus, a positional relationship is realized such that the image Im1 at a depth corresponding to the blood-vessel region Mp in the subject portion Mp0 irradiated with mid-infrared light is transferred onto the screen 30a of the photodetector 30 as the image Im2 of equivalent size.

Here, for example, the blood-vessel region Mp is located in a portion of the living body that is located inward of the epidermis (also referred to hereinafter as "a portion of the living body inwards of the surface of the skin" or "an inwards portion of the living body"), such as the dermis for example. In the present embodiment, for example, the depth corresponding to the blood-vessel region Mp may be 0.5-3.5 mm, inclusive, and may be 1.5 mm, for example. Here, the distances Op21 and Op22 may both be 2f, where f is the focal length of focal points Fp of the imaging lens 40.

However, the lengths of the distances Op21 and Op22 are not limited to those described above; the factors applied to the distances Op21 and Op22 may be set so that the image Im1 irradiated with mid-infrared light fits precisely in the screen 30a of the photodetector 30, and an imaging lens 40 achieving such factors may be configured.

The incident angle of the signal light L2 on the imaging lens 40 is determined by the angle of the optical axis of the signal light L2 passing through the center of the imaging lens 40 with respect to the normal to the portion of the surface of the skin of the subject portion Mp0 from which the signal light L2 is emitted. The angle of the optical axis passing through the center of the imaging lens 40 is equal to the above-described installation angle B of the photodetector 30 relative to the surface of the skin of the living body Ob, and may be set to 0-40 degrees, inclusive, and more preferably 20-30 degrees, inclusive, for example, in the present embodiment.

### (Light Detection Unit 70)

In the substance-in-blood concentration measurement device 1, in a state in which the second laser beam for reference measurement is emitted from the light emission unit 20, the photodetector 30 is moved along the optical path Op2 of the signal light L2 from the subject portion Mp0 to the photodetector 30 to change the distance of the photodetector 30 from the imaging lens 40. Furthermore, the positional relationship between the laser-beam condensing region FA and the photodetector 30 in the direction perpendicular to the optical path Op2 of the signal light L2 from the laser-beam condensing region FA in the subject portion Mp0 to the photodetector 30 can be adjusted by moving the photodetector 30 in a direction that intersects the optical path Op2 in a predetermined angle, such as a substantially perpendicular direction, for example. Here, the photodetector 30 may be moved in the direction along the optical path Op1 of the laser beam L1.

### (Photodetector 30)

The photodetector 30 is a near-infrared/mid-infrared sensor that receives the signal light L2 emitted from the laser-beam condensing region FA based on the emitted laser beam L1, and detects the intensity of the signal light L2. The photodetector 30 outputs an electric signal corresponding to the intensity of the received signal light L2. For example, as the photodetector 30, a single-element infrared sensor that outputs the intensity of the signal light L2 as a one-dimensional voltage value may be used.

In the device 1, the photodetector 30 can receive signal light having sufficiently high intensity relative to background light as a result of the intensity of the emitted laser beam L1 being increased by the light emission unit 20 and the signal light L2 from the laser-beam condensing region FA being imaged as a focused image on the screen 30a of the photodetector 30 by the imaging lens 40; thus, a high signal-to-noise (S/N) ratio is realized and measurement can be performed with high accuracy.

Because the laser beam L1 and the signal light L2 are monochromatic and have high intensity as discussed above, the photodetector 30 only needs to perform processing of detecting light intensity, and does not need to execute multivariate analysis, spectrum analysis, etc., based on wavelength sweeping as in a photoacoustic optical method in which a quantum cascade laser is used. Thus, the accuracy required in the detection is relaxed, and the easily usable electronic cooling method, etc., can also be used.

Note that, as the photodetector 30, an HgCdTe infrared photodetector cooled with liquid nitrogen may be used, for example. In this case, the light intensity of the signal light L2 can be detected with a higher S/N ratio by cooling the photodetector 30 to approximately 77 K using liquid nitrogen.

The photodetector 30 is electrically connected to the later-described measurement controller 60, and, based on a control signal from the measurement controller 60, outputs the intensity of received signal light to the measurement controller 60 as a one-dimensional voltage value.

### [Movable Mechanism 71]

The movable mechanism 71 is constituted from a first linear transportation mechanism 711 that can reversibly move the photodetector 30 along the optical path Op2 of the signal light L2 from the subject portion Mp0 to the photodetector 30, and a second linear transportation mechanism 712 that can reversibly move the photodetector 30 in the direction perpendicular to the optical path Op2.

By the function of the first linear transportation mechanism 711, the position of the photodetector 30 parallel to the optical path Op2 from the photodetector 30 to the signal light L2 can be adjusted so that the image Im1 at the depth corresponding to the blood-vessel region Mp in the subject portion Mp0 is transferred onto the screen 30a of the photodetector 30 as the image Im2 of equivalent size. Furthermore, the first linear transportation mechanism 711 may reversibly move the imaging lens 40 along the optical path Op2 together with the photodetector 30.

Furthermore, by the function of the second linear transportation mechanism 712, the position of the photodetector 30 can be adjusted so that the center of the screen 30a of the photodetector 30 and the position (laser-beam condensing region FA) in the subject portion Mp0 to which the laser beam L1 is condensed and emitted are substantially aligned or overlap in the direction perpendicular to the optical path Op2 of the signal light L2 to the photodetector 30.

In each of the first linear transportation mechanism 711 and the second linear transportation mechanism 712, a general-purpose linear transportation mechanism such as a linear motor, a lead screw, a ball screw, or a rack-and-pinion mechanism can be used. Alternatively, without using the linear transportation mechanisms 711 and 712, a reflection mechanism (mirror) including a micro-electromechanical systems (MEMS) actuator in which a piezoelectric element is used, etc., may be inserted into the laser optical path to change the laser optical path and switch to the correct detector position. The movable mechanism 71 is electrically connected to the later-described measurement controller 60, and, based on a control signal supplied from the measurement controller 60, transports the photodetector 30 to a predetermined position.

For example, the first linear transportation mechanism 711 is driven based on a control signal from the measurement controller 60, and actuates a linear motion mechanism to change the position in the direction parallel to the optical path Op2 to the photodetector 30 and also outputs position information regarding the position along the direction to the measurement controller 60. Similarly, the second linear transportation mechanism 712 is driven based on a control signal from the measurement controller 60, and actuates a linear motion mechanism to change the position in the direction perpendicular to the optical path Op2 to the photodetector 30 and also outputs position information regarding the position along the direction to the measurement controller 60.

Furthermore, in the device 1, the operation of changing the position in the direction perpendicular to the optical path Op2 to the photodetector 30 by means of the second linear transportation mechanism 712 may be performed together with the operation of changing the height ht in the direction perpendicular to the main surface 10a by means of the height adjustment mechanism 12. Thus, the emission position of the laser beam L1 can be changed and adjusted in the direction parallel to the center line of the finger on the skin surface on the back side of the finger. This will be described in detail later.

### (Measurement Controller 60)

The measurement controller 60 is electrically connected to the light emission unit 20, the photodetector 30, and the movable mechanism 71. The measurement controller 60 is circuitry that drives the light source 21 of the light emission unit 20 to cause the light source 21 to oscillate the pulsed pump light L0, and detects the light intensity of the signal light L2 based on an output signal from the photodetector 30 to calculate the concentration of a substance in blood in the blood-vessel region Mp of the subject portion Mp0.

Also, here, the output from the photodetector for monitoring may be input to the measurement controller 60, and, even if the intensity of the laser beam L1 emitted from the light emission unit 20 changes, the measurement controller 60 may calculate the concentration of a substance in blood while compensating for the influence of the change in the intensity of the laser beam L1 by normalizing the output from the photodetector 30 using the output from the photodetector for monitoring, as described above.

Furthermore, the measurement controller 60 is electrically connected to the rotation stage mechanism 11 and the height adjustment mechanism 12 of the base 10, and functions as control circuitry that controls these mechanisms. That is, the measurement controller 60 outputs a control signal to the base 10 to drive the rotation stage mechanism 11 of the base 10 and adjust the incident angle A relative to the subject portion Mp0, and to also drive the height adjustment mechanism 12 of the base 10 to change the height ht in the direction perpendicular to the main surface 10a and adjust the position in the subject portion Mp0 to which the laser beam L1 is emitted.

Furthermore, the measurement controller 60 is electrically connected to the movable mechanism 71, and functions as control circuitry that drives the first linear transportation mechanism 711 and the second linear transportation mechanism 712. That is, the measurement controller 60 outputs a control signal to the movable mechanism 71 to drive the first linear transportation mechanism 711 and transport the photodetector 30 to a predetermined position along the optical path Op2 of the signal light L2, and to also drive the second linear transportation mechanism 712 and transport the photodetector 30 to a predetermined position in the direction perpendicular to the optical path Op2 of the signal light L2. Furthermore, the first linear transportation mechanism 711 may reversibly move the imaging lens 40 along the optical path Op2 together with the photodetector 30.

For example, the measurement controller 60 may include a controller (unillustrated) with an internal central processing unit (CPU), and a data storage unit (unillustrated).

The data storage unit includes a volatile memory such as a dynamic random access memory (DRAM), for example, and a nonvolatile memory such as a hard disk, for example. An output signal acquired by the photodetector 30 is transmitted and stored to the data storage unit. Furthermore, information regarding the angle θC output from the rotation stage mechanism 11, information regarding the height ht output from the height adjustment mechanism 12, position information of the position of the photodetector 30 in the direction parallel to the optical path Op2 output from the first linear transportation mechanism 711, and position information of the position of the photodetector 30 in the direction perpendicular to the optical path Op2 output from the second linear transportation mechanism 712 are transmitted and stored to the data storage unit. Furthermore, the concentration of a substance in blood calculated by the controller may be stored to the data storage unit. Furthermore, the data storage unit stores therein a program, etc., that are necessary for the execution of the functions of the device 1, and also has a function as a temporary storage area for temporarily storing results of calculation by the controller.

The CPU realizes the functions of the device 1 by reading and executing the program from the data storage unit.

Thus, the measurement controller 60 executes the later-described substance-in-blood concentration measurement processing based on a predetermined program and executes the above-described processing, such as the transportation of the photodetector 30, the emission of the laser beam L1 from the light emission unit 20, and the calculation of the concentration of a substance in blood based on a signal from the photodetector 30.

### <Effects Achieved by Substance-In-Blood Concentration Measurement Device 1>

### (Realization of Structure for Performing Accurate Measurement Stably by Laser Emission from Main Surface 10a-Side>

According to the above-described structure, the device 1 can perform accurate measurement stably with a structure in which the concentration of a substance in blood is measured by emitting the laser beam L1 to the subject portion Mp0 of the living body Ob from the main surface 10a-side of the base 10, on which the living body Ob is placed, and receiving reflected light based on the laser beam L1 on the main surface 10a-side.

Specifically, in the device 1, the position of the light emission unit 20 relative to the main surface 10a of the base 10 is regulated, and, simultaneously, the position of the photodetector 30 relative to the main surface 10a of the base 10 and the light emission unit 20 is regulated via the movable mechanism 71. Thus, in the device 1, the installation angle B can be set to the predetermined angle θB in a state in which the incident angle A is equal to the predetermined angle θA.

Furthermore, due to the device 1 including the rotation stage mechanism 11, which changes the angle θC of the main surface 10a of the base 10, the incident angle A and the installation angle B relative to the subject portion Mp0 can be adjusted simultaneously in accordance with the shape or size of the individual living body Ob even if the shape of the surface of the skin of the living body Ob differs depending on the subject person.

Furthermore, due to the device 1 including the height adjustment mechanism 12, which can adjust the height ht of the main surface 10a of the base 10, a change in the distance from the main surface 10a to the subject portion Mp0 present in the skin on the back side of the finger serving as the living body Ob due to the thickness of the finger serving as the living body Ob can be absorbed, and a difference in the thickness of the finger serving as the living body Ob, which differs depending on the subject person, can be absorbed.

Due to this, in a state in which the height ht has been adjusted so as to be suitable for the subject person, the position in the subject portion Mp0 of the living body Ob to which the laser beam L1 is emitted, the incident angle A of the laser beam L1 relative to the subject portion Mp0, and the installation angle B of the photodetector 30 relative to the subject portion Mp0 can be regulated to near the appropriate values necessary for measurement.

Thus, a fluctuation in measurement results caused by a difference in the manner in which a measurement-target part is placed on a device, which is experienced with a structure in which measurement is performed by placing a measurement-target part into contact with a light-emission window, can be suppressed, and accurate measurement can be stably performed.

### (Improvement of S/N ratio)

An improvement in S/N ratio during measurement achieved by the optical system in the device 1 will be described.

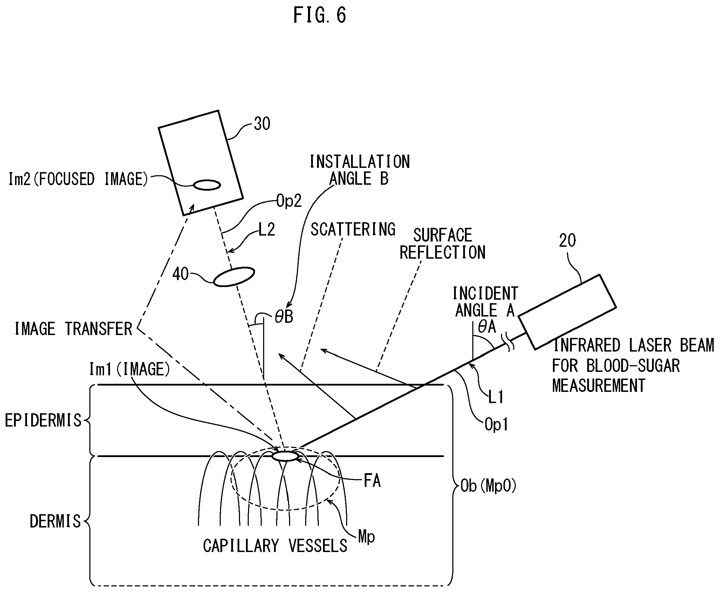

FIG. 6 is a schematic diagram illustrating an overview of an optical path from the light emission unit 20 to the photodetector 30 in the device 1.

As illustrated in FIG. 6, in the device 1, the laser beam L1 condensed and emitted from the light emission unit 20 enters the living body Ob at the incident angle A (angle θA) along the optical path Op1 toward the specific region (laser-beam condensing region FA) in the portion of the living body inwards of the surface of the skin in the subject portion Mp0, and is absorbed by substances in blood in the blood-vessel region Mp located in the portion of the living body inwards of the surface of the skin, resulting in an image (Im1) being formed of the signal light L2 from the portion of the living body inwards of the surface of the skin (laser-beam condensing region FA) having lower light intensity than the laser beam L1 in some wavelengths. Additionally, surface reflection from the surface of the skin is also produced.

In the device 1, a focused image is formed by the imaging lens 40 on the screen 30a of the photodetector 30 mainly from the image (Im1) of the signal light L2 from the laser-beam condensing region FA in the portion of the living body inwards of the surface of the skin, among the image (Im1) of the signal light L2 and the surface reflection from the surface of the skin. For example, the positional relationship between the laser-beam condensing region FA, the imaging lens 40, and the photodetector 30 is set so that the relationship 1/f = 1/a + 1/b holds true, where f is the focal length of the imaging lens 40, a is an optical distance OP21 between the laser-beam condensing region FA (blood-vessel region Mp) and the imaging lens 40, and b is an optical distance OP22 between the screen 30a of the photodetector 30 and the imaging lens 40.

Thus, by disposing the screen 30a of the photodetector 30 at a position where the optical distance OP22 equals b, the image (Im1) of the signal light L2 from the laser-beam condensing region FA is clearly imaged (forms a focused image) on the screen 30a, and a relatively high signal intensity value is detected by the photodetector 30.

On the other hand, the light that is surface-reflected from the surface of the skin enters the imaging lens 40. However, because the incident angle of the surface-reflected light on the imaging lens 40 is different from that of the signal light L2 from the laser-beam condensing region FA, the surface-reflected light is guided to the outside of the area of the screen 30a of the photodetector 30. Otherwise, even if the surface-reflected light is guided to within the area of the screen 30a of the photodetector 30, the surface-reflected light is not focused (is blurred). Thus, the light amount thereof decreases and the signal intensity detected by the photodetector 30 decreases.

That is, because the epidermis is slightly closer to the imaging lens 40 than the blood-vessel region Mp is, the image transfer position is located at a position b' satisfying 1/f = 1/a' + 1/b', where a' is the optical distance OP21 between the epidermis and the imaging lens 40. Thus, the image from the epidermis is blurred and not clear (not focused) on the screen 30a, which is located at position b, and therefore the signal intensity detected by the photodetector 30 relatively decreases.

Due to this, the surface reflection from the surface of the skin, which is detected as noise, does not affect the optical measurement much.

Thus, in the device 1, the image (Im1) of the signal light L2 from the laser-beam condensing region FA that is to be mainly measured and located in the blood-vessel region Mp (inwards portion of the living body) inwards of the surface of the skin is transferred by the imaging lens 40 so as to form a focused image on the screen 30a of the photodetector 30 and is reflected in the optical measurement by the photodetector 30. Thus, a measurement result that is highly correlated with a blood-sugar-level measurement result based on self-monitoring-of-blood-glucose (SMBG) and that has high reproducibility can be obtained.

If a lens that is merely for condensing diffused light (scattered light) were installed and the photodetector 30 were disposed near the focal point of the lens, the signal light from the blood-vessel region Mp (inwards portion of the living body) would be equivalent to scattered light, making it difficult to distinguish the signal light from scattered light from the skin surface portion. In contrast, the light amount of scattered light from the epidermis portion can be relatively reduced and the S/N ratio of the signal light L2 can be improved by installing structures such as the imaging lens 40 and the photodetector 30 at positions where transfer of a focused image is possible and causing the structures to transfer a focused image of the signal light L2 from the blood-vessel region Mp (inwards portion of the living body).

In such a manner, a false-signal (noise) component produced by signal light scattered at the surface of the skin can be reduced and the S/N ratio in optical measurement can be improved with the device 1.

### (Detection of Blood-Vessel Region in Subject Portion Mp0)

An improvement in measurement accuracy based on the detection of the blood-vessel region Mp in the subject portion Mp0 using the optical system in the device 1 will be described.

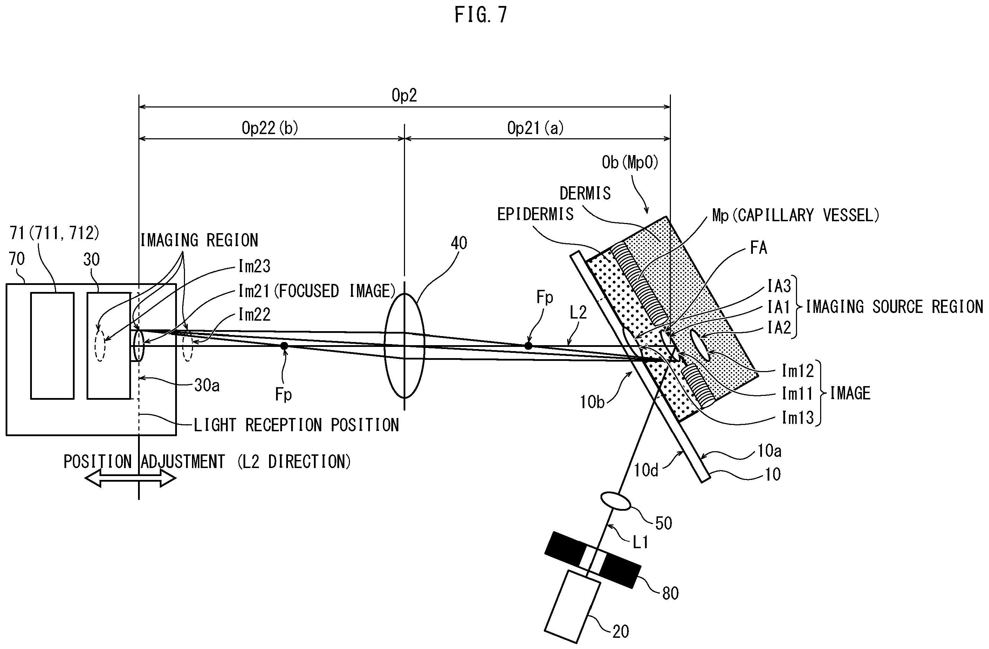

FIG. 7 is a schematic diagram for describing an operation by the device 1 for adjusting the length of the optical path from the laser-beam condensing region FA to the photodetector 30.

As illustrated in FIG. 7, in the device 1, in a state in which the laser beam L1 for reference measurement (second laser beam) is emitted from the light emission unit 20, the photodetector 30 is moved, by means of the first linear transportation mechanism 711 of the movable mechanism 71, along the optical path Op2 of the signal light L2 from the subject portion Mp0 to the photodetector 30 to change and adjust the distance of the photodetector 30 from the imaging lens 40.

According to this structure, a function is realized of changing an imaging source region (IA1, IA2, or IA3) in the subject portion Mp0 from which an image (Im11, Im12, or Im13) of signal light L2 forming a focused image on the screen 30a of the photodetector 30 is extracted.

Here, the imaging source region (IA1, IA2, or IA3) is the focus region in the subject portion Mp0 where the focal point Fp of the imaging lens 40 is in focus with.

For example, by disposing the photodetector 30 in the position illustrated in FIG. 7, an image Im11 of signal light L2 from an imaging source region A1 located in the blood-vessel region Mp in the subject portion Mp0 can be formed as a focused image (Im21) on the screen 30a. Because the imaging source region IA1 is located in a region where the laser-beam condensing region FA and the blood-vessel region Mp overlap, the image Im11 of the signal light L2 from the blood-vessel region Mp in the subject portion Mp0 can be formed as a focused image on the screen 30a by disposing the photodetector 30 in the position illustrated in FIG. 7.

Here, an image Im12 of signal light L2 from an imaging source region IA2 located in the dermis portion in the subject portion Mp0 forms a focused image (Im22) in front of the screen 30a. Thus, the image (Im22) is blurred and not clear (not focused) on the screen 30a, and detected signal intensity relatively decreases.

Furthermore, similarly, an image Im13 of signal light L2 from an imaging source region IA3 located in the epidermis portion in the subject portion Mp0 forms an image behind the screen 30a. Thus, the image (Im23) is not clear (not focused) on the screen 30a, and the detected signal intensity relatively decreases.

By using this function and detecting signal light from a blood vessel based on signal light corresponding to the laser beam for reference measurement (second laser beam) while gradually moving the photodetector 30 along the optical path Op2 of the signal light L2 and detecting the position of the photodetector 30 where the intensity of the signal light increases, a state can be detected in which the imaging source region that is to become the measurement-target region for measuring the concentration of a substance in blood is located in a region where the blood-vessel region Mp and the laser-beam condensing region FA overlap in the living body, or that is, a state can be detected in which the imaging lens 40 is in focus with the region.

That is, the device 1 can detect a state in which the image Im11 based on the signal light L2 from the region where the laser-beam condensing region FA and the blood-vessel region Mp overlap is imaged as a focused image on the photodetector 30 by, in a state in which the laser beam L1 for reference measurement (second laser beam) is emitted, receiving the signal light L2 from the laser-beam condensing region FA and measuring the concentration of the reference substance (second substance in blood). The reference substance (second substance in blood) is characterized in that the reference substance (second substance in blood) has a higher laser-beam absorption rate than the measurement-target substance (first substance in blood) and/or the concentration in blood of the reference substance (second substance in blood) is more stable than that of the measurement-target substance (first substance in blood).

Accordingly, signal light from a blood vessel can be detected based on signal light corresponding to the laser beam for reference measurement (second laser beam) while gradually moving the photodetector 30.

### (Appropriate Measurement Position on Surface of Skin of Living Body Ob)

A reduction, achieved by the optical system of the device 1, of measurement fluctuation caused by the measurement position on the surface of the skin of the living body Ob will be described.

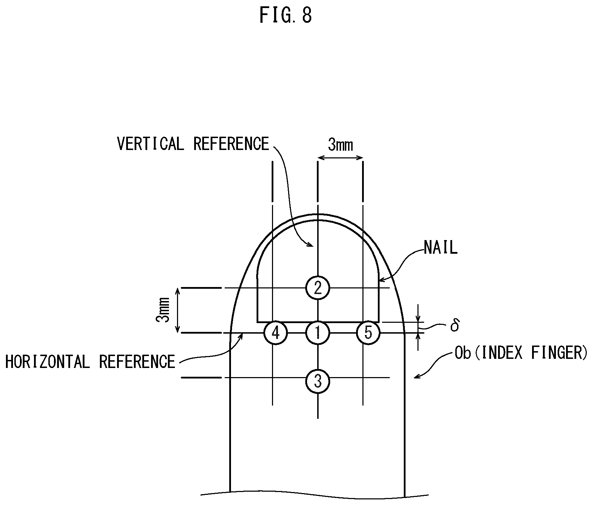

FIG. 8 is a diagram illustrating laser-beam emission positions on the surface of a living body in substance-in-blood-concentration measurement by the device 1. The index finger was used as the living body Ob. Among measurement position 1, which is a position on a center line toward the fingertip and a cutting line that is shifted by δ toward the finger-root direction from the root of the nail, measurement positions 2 and 3, which are positions on the center line and respectively offset by 3 mm upward and downward in the drawing, and measurement positions 4 and 5, which are positions on the cutting line and respectively offset by 3 mm toward the left and right in the drawing, a blood-vessel region Mp in which a laser beam is absorbed by hemoglobin to a great extent and the hemoglobin concentration has a high value is present at measurement positions 4 and 5.

Furthermore, by emitting the laser beam for target measurement (first laser beam) from the light emission unit 20 at in-plane-direction measurement position 4 or 5, where signal light indicating a blood-vessel region Mp is detected, and receiving signal light at the measurement position using the photodetector 30, the concentration of the measurement-target substance can be measured in a state in which the in-plane-direction measurement position is included in a blood-vessel region Mp in the subject portion Mp0.

Thus, accurate measurement can be performed stably at all times regardless of differences between individuals in in-plane-direction blood-vessel positions, which differ between subject persons and instances of measurement.

As described above, in the device 1, the laser-beam condensing region FA in the surface plane of the living body during measurement is defined by setting the mark on the main surface 10a of the base 10 to a predetermined position in advance. Thus, by changing the position of the measurement-position mark on the main surface 10a in advance, the laser-beam condensing region FA in the surface plane of the living body during measurement can be adjusted. For example, in the device 1, the position of the mark on the main surface 10a of the base 10 may be set in advance so that a laser beam is emitted to measurement position 4 or 5 during measurement. Thus, accurate measurement can be performed stably at all times in a state in which a blood-vessel region Mp is the measurement target.

Furthermore, in the device 1, the emission position of the laser beam L1 may be changed in a direction (also referred to hereinafter as "finger center line direction") that is parallel to the center line of the finger on the skin surface on the back side of the finger by combining the operation of changing the height ht in the direction perpendicular to the main surface 10a by means of the height adjustment mechanism 12 and the operation of changing the position in the direction perpendicular to the optical path Op2 of the signal light L2 to the photodetector 30 by means of the second linear transportation mechanism 712.

Specifically, for example, when the height ht of the main surface 10a is increased in a state in which the palm side of the finger serving as the living body Ob is positioned with respect to the mark on the main surface 10a, the laser-beam condensing region FA moves toward the tip direction of the finger. Thus, the photodetector 30 is moved in a direction that is toward the light emission unit 20 and perpendicular to the optical path Op2 to the photodetector 30 by means of the second linear transportation mechanism 712 so that the laser-beam condensing region FA and the center of the screen 30a of the photodetector 30 are substantially aligned or overlap in the direction perpendicular to the optical path Op2 to the photodetector 30.

Furthermore, for example, when the height ht of the main surface 10a is conversely decreased in the same state, the laser-beam condensing region FA moves toward the root direction of the finger. Thus, the photodetector 30 is moved in a direction that is away from the light emission unit 20 and perpendicular to the optical path Op2 by means of the second linear transportation mechanism 712 so that the laser-beam condensing region FA and the center of the screen 30a of the photodetector 30 are substantially aligned or overlap in the direction perpendicular to the optical path Op2 of the signal light L2.

Furthermore, in the measurement controller 60, a height ht of the main surface 10a and a position in the direction perpendicular to the optical path Op2 may be stored in advance in the data storage unit or the like as a pair of combination information. Based on this information, the measurement controller 60 may change the laser-beam condensing region FA in the finger center line direction by performing in parallel the operation of changing the height ht by means of the height adjustment mechanism 12 and the operation of changing the position of the photodetector 30 by means of the second linear transportation mechanism 712.

### <Operation of Substance-In-Blood Concentration Measurement Device 1>

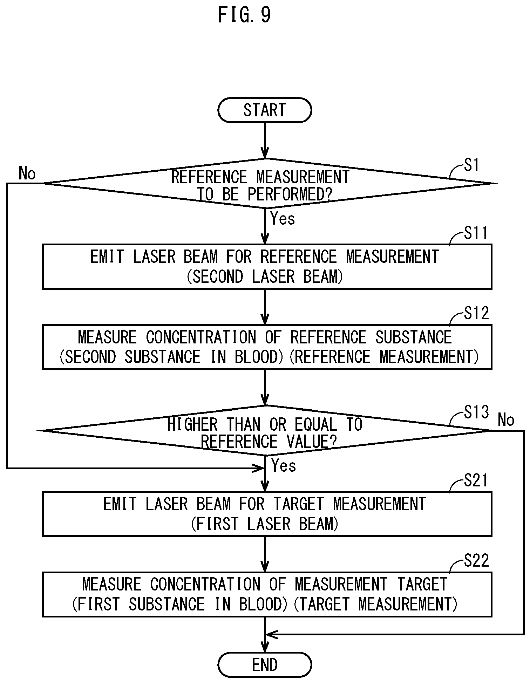

An outline of operation of the device 1 will be described with reference to FIGS. 9, 10, and 11.

### (Operation for Determining Whether or Not Laser-Beam Condensing Region FA Is Included in Blood-Vessel Region Mp in Subject Portion Mp0)

FIG. 9 is a flowchart illustrating one aspect of a substance-in-blood measurement operation by the device 1.

In FIG. 9, steps S11 and S12 are steps for performing the reference measurement, in which the concentration of the reference substance (second substance in blood) in the specific region (laser-beam condensing region FA) to which the laser beam L1 is condensed and emitted is measured to determine whether or not the laser-beam condensing region FA is included in a blood-vessel region Mp in the subject portion Mp0.

Steps S21 and S22 are steps for the target measurement, in which the concentration of the measurement-target substance (first substance in blood) is measured in a state in which the laser-beam condensing region FA is included in a blood-vessel region Mp in the subject portion Mp0.

In FIG. 9, first, it is determined whether or not the reference measurement is to be performed (step S1). This determination may be carried out based on input of an operation by an operator or identification information such as the ID of the subject person. For example, in a case in which the same subject person is measured repetitively on a daily basis, reference measurement results that have already been acquired can be used, and thus the reference measurement can be skipped. Processing proceeds to step S11 if the result of the determination in step S1 is that the reference measurement is to be performed (step S1: Yes), and processing proceeds to step S21 if the result of the determination in step S1 is that the reference measurement is not to be performed (step S1: No).

Next, the reference measurement is performed in steps S11 and S12. First, based on a control signal, the measurement controller 60 condenses and emits the laser beam for reference measurement (second laser beam) from the light emission unit 20 to the specific region in the subject portion Mp0 (step S11), measures the concentration of the reference substance (second substance in blood) in the laser-beam condensing region FA by means of the photodetector 30 (step S12), and determines whether or not the measured concentration of the reference substance is higher than or equal to a reference value (step S13).

Processing is terminated if the measured concentration is not higher than or equal to the reference value in step S13 (step S13: No), whereas the concentration of the measurement-target substance (first substance in blood) is measured in steps S21 and S22 if the measured concentration is higher than or equal to the reference value in step S13 (step S13: Yes) because it is determined that the laser-beam condensing region FA is included in a blood-vessel region Mp in the subject portion Mp0.

Specifically, the laser beam for target measurement (first laser beam) is emitted from the light emission unit 20 to the specific region in the subject portion Mp0 (step S21), a measurement of the concentration of the measurement-target substance in the laser-beam condensing region FA (target measurement) is performed by means of the photodetector 30 to output a measurement result (step S22), and processing is terminated.

### (Operation for Adjusting Photodetector Position along Optical Path)

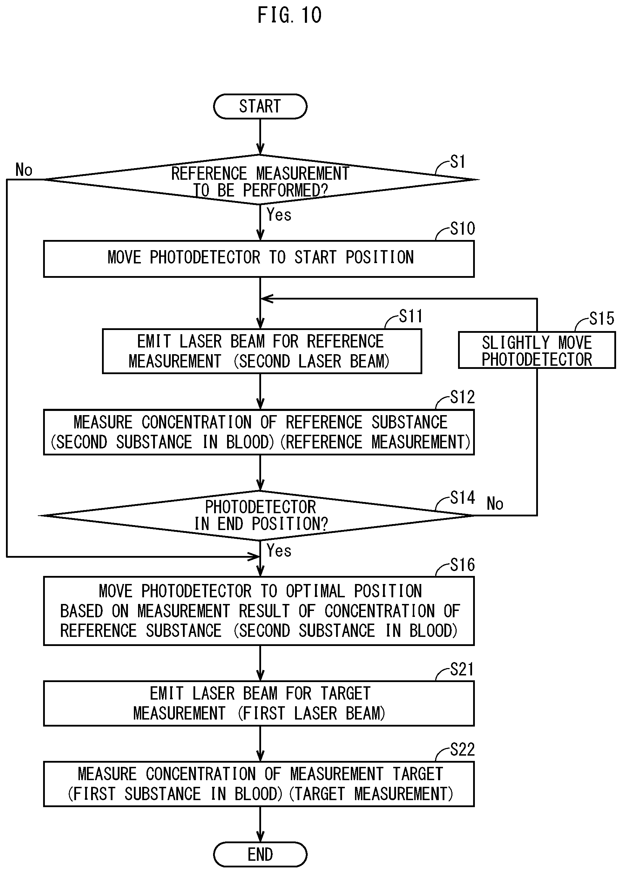

FIG. 10 is a flowchart illustrating another aspect of the substance-in-blood measurement operation by the substance-in-blood concentration measurement device 1.

In FIG. 10, steps S10 to S16 are steps for measuring the concentration of the reference substance (second substance in blood) in the specific region to which the laser beam L1 is condensed and emitted as the reference measurement, and adjusting the position of the photodetector along the optical path Op2 based on the concentration of the reference substance in the laser-beam condensing region FA so that the laser-beam condensing region FA is included in a blood-vessel region Mp in the subject portion Mp0.

Steps S21 and S22 are steps for the target measurement, in which the concentration of the measurement-target substance (first substance in blood) is measured in a state in which the laser-beam condensing region FA is included in a blood-vessel region Mp in the subject portion Mp0. Processing that is the same as processing in FIG. 9 is indicated with the same number as that in FIG. 9.

In FIG. 10, if the result of the determination in step S1 is that the reference measurement is to be performed (step S1: Yes), the reference measurement is performed in steps S10 to S16. First, based on a control signal, the measurement controller 60 drives the first linear transportation mechanism 711 of the movable mechanism 71 to move the photodetector 30 to a start position (step S10), condenses and emits the laser beam for reference measurement (second laser beam) from the light emission unit 20 to the specific region in the subject portion Mp0 (step S11), and measures the concentration of the reference substance (second substance in blood) in the laser-beam condensing region FA by means of the photodetector 30 (reference measurement; step S12). Here, the measurement controller 60 detects the light intensity of the signal light L2 based on an output signal from the photodetector 30, and calculates and stores the concentration of the reference substance in the laser-beam condensing region FA. At the same time, the measurement controller 60 stores position information of the optical path Op2-direction position of the photodetector 30 in the first linear transportation mechanism 711.

Next, it is determined whether or not the photodetector 30 is in an end position (step S14). If the photodetector 30 is not in the end position (step S14: No), the position of the photodetector 30 is moved slightly (step S15), and processing returns to step S11, whereas, if the photodetector 30 is in the end position (step S14: Yes), processing proceeds to step S16, it being regarded that the measurement of the concentration of the reference substance in the laser-beam condensing region FA has been performed at all positions of the photodetector 30. In this state, position information of the photodetector 30 at all positions of the photodetector 30 and the concentration of the reference substance in the laser-beam condensing region FA at all positions of the photodetector 30 have been stored in the measurement controller 60.

In step S16, based on the results of the measurement of the concentration of the reference substance in the laser-beam condensing region FA at all positions of the photodetector 30, the measurement controller 60 selects an optimal position where the result indicating the highest reference-substance concentration was obtained, and drives the first linear transportation mechanism 711 and moves the photodetector 30 to the optimal position by transmitting a control signal to the movable mechanism 71. It can be inferred that the laser-beam condensing region FA is included in a blood-vessel region Mp in the subject portion Mp0 at the optimal position. Note that, also in a case in which the result of the determination in step S1 is that the reference measurement is not to be performed (step S1: No), the photodetector 30 is moved to a predetermined optimal position, for example, in step S16.

Next, the concentration of the measurement-target substance (first substance in blood) is measured in steps S21 and S22. Specifically, the laser beam for target measurement (first laser beam) is condensed and emitted from the light emission unit 20 to the specific region in the subject portion Mp0 (step S21), a measurement of the concentration of the measurement target in the laser-beam condensing region FA is performed by means of the photodetector 30 to output a measurement result (target measurement, step S22), and processing is terminated.

### (Operation for Adjusting In-Plane-Direction Emission Position of Laser Beam L1)

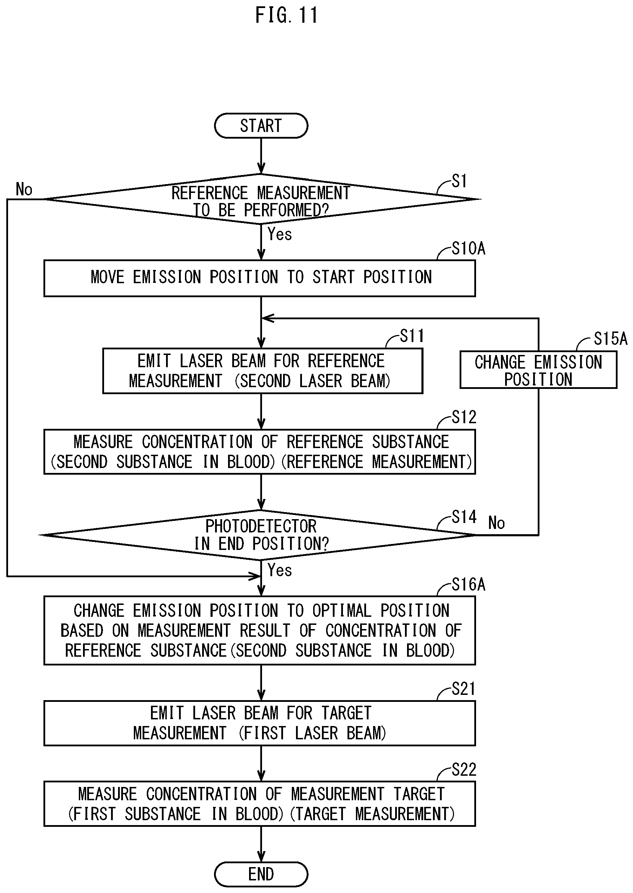

FIG. 11 is a flowchart illustrating yet another aspect of the substance-in-blood measurement operation by the substance-in-blood concentration measurement device 1.

In FIG. 11, steps S10A to S16A are steps for measuring the concentration of the reference substance (second substance in blood) in the specific region while changing the in-plane direction position in the subject portion Mp0 to which the laser beam L1 is condensed and emitted as the reference measurement, and, based on the concentration of the reference substance at different emission positions, adjusting the in-plane-direction emission position so that the emission position is included in a blood-vessel region Mp in the in-plane direction in the subject portion Mp0. In the present example, a structure will be described in which the emission position of the laser beam L1 is changed in the finger center line direction as an adjustment of the in-plane direction position of the emission position of the laser beam L1.

Steps S21 and S22 are steps for the target measurement, in which the concentration of the measurement-target substance (first substance in blood) is measured in a state in which the emission position is included in a blood-vessel region Mp in the in-plane direction in the subject portion Mp0.

Processing that is the same as processing in FIGS. 9 and 10 is indicated with the same number.

In FIG. 11, if the result of the determination in step S1 is that the reference measurement is to be performed (step S1: Yes), the reference measurement is performed in steps S10A to S16A. First, based on a control signal, the measurement controller 60 moves the in-plane-direction position to which the laser beam L1 is emitted, which is the finger center line direction position in this example, to a start position (step S10A), condenses and emits the laser beam for reference measurement (second laser beam) from the light emission unit 20 to the specific region in the subject portion Mp0 (step S11), and measures the concentration of the reference substance (second substance in blood) in the laser-beam condensing region FA by means of the photodetector 30 (step S12). Here, in the device 1, the emission position of the laser beam L1 is changed in the finger center line direction by performing, in combination, the operation of changing the height ht in the direction perpendicular to the main surface 10a by means of the height adjustment mechanism 12 and the operation of changing the position in the direction perpendicular to the optical path Op2 by means of the second linear transportation mechanism 712.

First, the measurement controller 60 detects the light intensity of the signal light L2 based on an output signal from the photodetector 30 at the start position, and calculates and stores the concentration of the reference substance in the laser-beam condensing region FA. At the same time, the measurement controller 60 stores information of the height ht of the main surface 10a in the height adjustment mechanism 12 and position information of the position of the photodetector 30 in the direction perpendicular to the optical path Op2 in the second linear transportation mechanism 712.

Next, it is determined whether or not the emission position is in an end position (step S14). If the emission position is not in the end position (step S14: No), the emission position is changed (step S15A), and processing returns to step S11, whereas, if the emission position is in the end position (step S14: Yes), processing proceeds to step S16A, it being regarded that the measurement of the concentration of the reference substance in the laser-beam condensing region FA has been performed at all emission positions. In this state, position information of the photodetector 30, information of the height ht of the main surface 10a, and the concentration of the reference substance in the laser-beam condensing region FA at all emission positions of the laser beam L1 in the finger center line direction have been stored in the measurement controller 60.

In step S16A, based on the results of the measurement of the concentration of the reference substance (second substance in blood) in the laser-beam condensing region FA at all emission positions, the measurement controller 60 selects an optimal emission position where the result indicating the highest reference-substance concentration was obtained. Furthermore, the measurement controller 60 changes the emission position of the laser beam L1 to the optimal position by transmitting a control signal to drive the height adjustment mechanism 12 to change the height ht of the main surface 10a of the base 10 to a height corresponding to the optimal emission position and also transmitting a control signal to the movable mechanism 71 to drive the second linear transportation mechanism 712 and move the photodetector 30 to a position corresponding to the optimal emission position. It can be inferred that the emission position is included in a blood-vessel region Mp in the in-plane direction of the subject portion Mp0 at the optimal position. Note that, also in a case in which the result of the determination in step S1 is that the reference measurement is not to be performed (step S1: No), the emission position of the laser beam L1 is moved to a predetermined optimal position, for example, in step S16A.

Next, the concentration of the measurement-target substance (first substance in blood) is measured in steps S21 and S22. Specifically, the laser beam for target measurement (first laser beam) is condensed and emitted from the light emission unit 20 to the specific region in the subject portion Mp0 (step S21), a measurement of the concentration of the measurement target in the laser-beam condensing region FA is performed by means of the photodetector 30 to output a measurement result (target measurement, step S22), and processing is terminated.

### <Conclusion>

As described above, a substance-in-blood concentration measurement device 1 according to Embodiment 1 is a substance-in-blood concentration measurement device 1 that measures a concentration of a substance in blood included in the blood in a subject portion Mp0 of a living body Ob, the substance-in-blood concentration measurement device 1 characterized by including: a base 10 having a main surface 10a on which the living body can be placed; a light emission unit 20 that, from the main surface 10a-side of the base 10, condenses and emits a laser beam L1 to a specific region in the subject portion Mp0, the specific region being present in skin located on the reverse side of the living body Ob from a skin surface on a side of the living body facing the main surface 10a; a photodetector 30 that, on the main surface 10a-side of the base 10, receives signal light that is reflected light based on the laser beam L1, and detects an intensity of the signal light, the signal light having lower light intensity than the laser beam L1 in some wavelengths; an imaging lens 40 that is disposed in a position between the subject portion Mp0 and the photodetector 30 where a focused image of the signal light from a laser-beam condensing region FA in the subject portion Mp0 can be formed on the photodetector 30; and a measurement controller that measures a concentration of the substance in blood in the laser-beam condensing region FA based on the intensity of the signal light, wherein a first angle formed by a normal to the surface of the skin of the subject portion Mp0 and an optical path Op1 of the laser beam L1 is different from a second angle formed by the normal and an optical path Op2 from the laser-beam condensing region FA to the photodetector 30, the position of the subject portion Mp0 in the living body Ob relative to the light emission unit 20 is set so that the laser-beam condensing region FA and a blood-vessel region Mp inwards of the epidermis in the subject portion Mp0 overlap, and the position of the photodetector 30 relative to the subject portion Mp0 in the living body Ob is set so that an image of the signal light from the blood-vessel region Mp overlapping the laser-beam condensing region FA is transferred by the imaging lens 40 to form a focused image on a light receiving surface of the photodetector.

Conventional substance-in-blood concentration measurement devices have a structure for performing measurement by placing a measurement-target part into contact with a light-emission window provided on the outer surface of the measurement device. Thus, there is a problem that it is difficult to stably obtain sufficient measurement accuracy because measurement results may fluctuate as a result of a change in pressure applied to the measurement-target part, measurement position, etc., caused by a difference in the manner in which the measurement-target part is placed on the light-emission window.

In contrast to this, according to the above-described structure of the device 1, a fluctuation in measurement results caused by a difference in the manner in which a measurement-target part is placed on the device can be suppressed and accurate measurement can be stably performed by, from the main surface 10a-side of the base 10, condensing and emitting a laser beam L1 to a specific region in a subject portion Mp0 that is present in the skin located on the reverse side of a living body Ob from a skin surface on a side of the living body facing the main surface 10a, and, on the main surface 10a-side of the base 10, receiving and measuring reflected light based on the laser beam L1, i.e., signal light having lower light intensity than the laser beam L1 in some wavelengths.

Furthermore, the base 10 may be capable of adjusting the position of the living body Ob relative to the light emission unit 20 by changing the height of the main surface 10a in a perpendicular direction.

According to this structure, the positional relationship of the living body Ob relative to the light emission unit 20 can be adjusted in accordance with the shape of the individual living body Ob so that the laser beam is condensed and emitted to the blood-vessel region Mp inwards of the epidermis in the subject portion Mp0. Thus, the position of the living body Ob relative to the light emission unit 20 is regulated so that the laser-beam condensing region FA and the blood-vessel region Mp inwards of the epidermis in the subject portion Mp0 overlap in an XZ plane.

Furthermore, the position of the photodetector 30 relative to the living body Ob in a direction intersecting the optical path Op2 of the signal light L2 at a predetermined angle such as a perpendicular angle, for example, may be capable of being adjusted by changing the position of the photodetector in said direction.

According to this structure, the position of the photodetector 30 can be adjusted so that the center of a screen 30a of the photodetector 30 and the position (laser-beam condensing region FA) in the subject portion Mp0 to which the laser beam L1 is condensed and emitted are substantially aligned or partially overlap in the direction perpendicular to the optical path Op2 to the photodetector 30.

Furthermore, the base 10 may be capable of adjusting the first angle and the second angle simultaneously by changing an angle of the main surface 10a relative to the optical path of the laser beam L1 within a plane that is defined by the optical path Op1 of the laser beam L1 and the optical path Op2 from the subject portion to the photodetector 30.

According to this structure, the incident angle A and the installation angle B relative to the subject portion Mp0 can be adjusted simultaneously in accordance with the shape of the individual living body Ob.

Furthermore, the position of the photodetector 30 relative to the living body Ob in a direction along the optical path Op2 of the signal light L2 may be capable of being adjusted by changing the position of the photodetector 30 in said direction, and the position of the photodetector 30 may be adjusted based on the concentration of the substance in blood so that the image of the signal light from the blood-vessel region Mp is transferred by the imaging lens 40 to form a focused image on the light receiving surface of the photodetector.

According to this structure, the position of the photodetector 30 in the direction parallel to the optical path Op2 can be adjusted so that an image Im1 at a depth corresponding to the blood-vessel region Mp in the subject portion Mp0 is transferred as an image Im2 of equivalent size on the screen 30a of the photodetector 30.

Due to this, the photodetector 30 can receive signal light having sufficiently high intensity relative to background light as a result of a focused image of the signal light L2 from the laser-beam condensing region FA being formed on the screen 30a of the photodetector 30 by the imaging lens 40. Thus, a high S/N ratio is realized and accurate measurement can be performed.

### <<Embodiment 2>>

A substance-in-blood concentration measurement device 1A according to Embodiment 2 will be described with reference to the drawings.

### (Configuration)

In above-described Embodiment 1, the base 10 includes the rotation stage mechanism 11 and the height adjustment mechanism 12, and the angle and height of the base 10 are adjusted so that the blood-vessel region Mp included in the subject portion Mp0 of the living body Ob is regulated to a predetermined position and angle suitable for the irradiation with the laser beam L1.

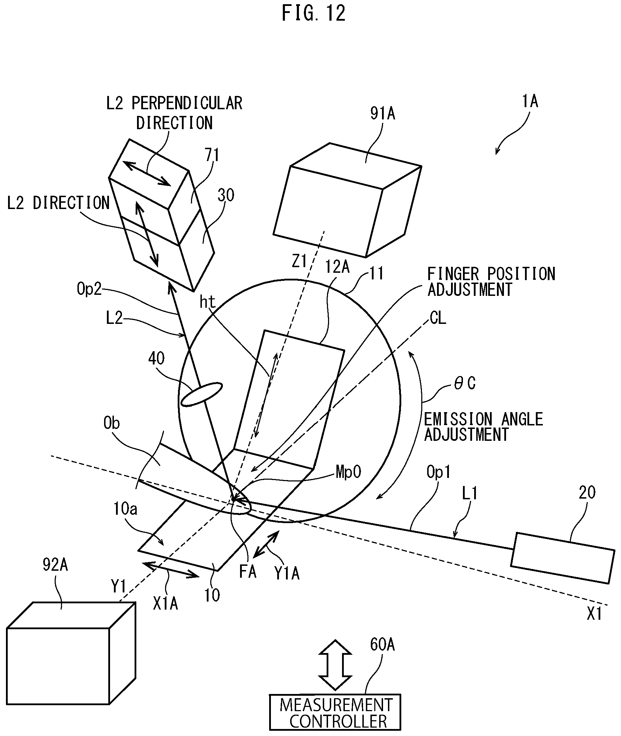

FIG. 12 is a schematic diagram illustrating a state of the substance-in-blood concentration measurement device 1A according to Embodiment 2 during measurement.

The substance-in-blood concentration measurement device 1A (also referred to hereinafter as "device 1A") according to Embodiment 2 has a structure including the base 10, the light emission unit 20, the photodetector 30, the imaging lens 40, a measurement controller 60A, the movable mechanism 71, the rotation stage mechanism 11, a position adjustment mechanism 12A, an image-capturing means 91A that is disposed in the direction of the normal to the main surface 10a of the base 10, and an image-capturing means 92A that is disposed in the direction parallel to the main surface 10a of the base 10.

The base 10 includes the position adjustment mechanism 12A. The position adjustment mechanism 12A can change the height ht in the direction perpendicular to the main surface 10a, and can also change the position (X1A, Y1A) in an in-plane direction (X1-Y1 direction) that is parallel to the main surface 10a.

Furthermore, as is the case in the device 1, the base 10 is mechanically coupled to the rotation stage mechanism 11. The rotation stage mechanism 11 changes the angle θC (see FIG. 3) of the main surface 10a relative to the optical path Op1 of the laser beam L1 and the optical path Op2 of the signal light L2 within the laser-beam incident plane. For example, the rotation stage mechanism 11 constitutes an angle changing mechanism that includes an internal motor and can reversibly rotate about a rotational axis CL that is parallel to the axis Y1. The rotational axis CL may intersect the position in space where the subject portion Mp0 is present.

The motor is driven based on a control signal from the measurement controller 60A, and the rotation stage mechanism 11 thereby actuates and rotates the angle changing mechanism to rotate the base 10 coupled to the rotation stage mechanism 11 about the rotational axis CL. Due to this, the incident angle A (see FIG. 3) of the laser beam L1 emitted from the light emission unit 20 on the subject portion Mp0 and the installation angle B (see FIG. 3) of the photodetector 30 relative to the subject portion Mp0 can be adjusted simultaneously. Thus, according to the rotation stage mechanism 11, the incident angle A and the installation angle B can be respectively set to the predetermined angles θA and θB (see FIG. 3) simultaneously.

The structures of the light emission unit 20, the photodetector 30, the imaging lens 40, and the movable mechanism 71 in the device 1A are the same as the structures of those in the device 1. The laser beam L1 emitted from the light emission unit 20 is emitted to the subject portion Mp0 of the living body Ob, and the signal light L2 reflected by the subject portion Mp0 is received by the photodetector 30.

Furthermore, also in the device 1A, as was the case in the device 1, the movable mechanism 71 is connected to the photodetector 30, and the position of the photodetector 30 relative to the base 10 can be changed. That is, by actuating the movable mechanism 71 based on a control signal from the measurement controller 60A, the photodetector 30 can be reversively moved in the direction parallel to the optical path Op2 of the signal light L2 from the laser-beam condensing region FA in the subject portion Mp0 to the photodetector 30. Similarly, the photodetector 30 can also be reversibly moved in the direction perpendicular to the optical path Op2 of the signal light L2. Furthermore, the movable mechanism 71 may move the imaging lens 40 together with the photodetector 30.

The image-capturing means 91A and the image-capturing means 92A are image-capturing means in which a charge coupled device (CCD) image sensor or the like is used, for example. The image-capturing means 91A and the image-capturing means 92A capture images of the living body Ob. The image-capturing means 91A is disposed in the direction of the normal to the main surface 10a of the base 10 so as to face the direction of the main surface 10a, and captures a plan-view image of the living body Ob placed on the main surface 10a. Furthermore, the image-capturing means 92A is disposed sideways of the main surface 10a of the base 10 so as to face the direction of the main surface 10a, and captures an image of the living body Ob placed on the main surface 10a as seen from sideways. Furthermore, image data captured by the image-capturing means 91A and the image-capturing means 92A is output to the measurement controller 60A.

The measurement controller 60A detects an image portion corresponding to the living body Ob from a received image, calculates a positional deviation amount from a reference position where the image portion should be located, and outputs a control signal for compensating for the positional deviation amount to the position adjustment mechanism 12A.

Specifically, for example, when a height deviation in the height direction is detected based on an image from the image-capturing means 92A, the position adjustment mechanism 12A compensates for the height deviation of the living body Ob by changing the height ht in the direction perpendicular to the main surface 10a based on a control signal. Similarly, for example, when a positional deviation in the in-plane direction is detected based on an image from the image-capturing means 91A, the position adjustment mechanism 12A compensates for the positional deviation of the living body Ob in the in-plane direction by changing the position (X1A, Y1A) of the base 10 in the in-plane direction (X1-Y1 direction) of the main surface 10a based on a control signal.

Alternatively, an angular deviation amount from a reference angle in which the image portion should be oriented may be calculated, a control signal for compensating for the angular deviation amount may be output to the position adjustment mechanism 12A, and the deviation of the angle θC may be compensated for by rotating the base 10 about the rotational axis CL by means of the rotation stage mechanism 11 coupled to the base 10.

Furthermore, in a state in which the laser beam L1 is emitted from the light emission unit 20 to the subject portion Mp0 of the living body Ob, the positions of the laser-beam condensing region FA and the subject portion Mp0 may be detected using images detected and captured by means of the image-capturing means 91A and 92A using a different wavelength from that of the laser beam L1, for example, and the rotation stage mechanism 11 and the position adjustment mechanism 12A may be driven based on the detection results to perform control of positions so that the subject portion Mp0 and the laser-beam condensing region FA are substantially aligned.

By detecting positions using the image-capturing means 91A and 92A, the subject portion Mp0 and the laser-beam condensing region FA can be substantially aligned with one another accurately.

### <Evaluation Test>

In the following, results obtained by evaluating performance through an evaluation test using an example of the device 1A and a comparative example will be described.

### (Example)

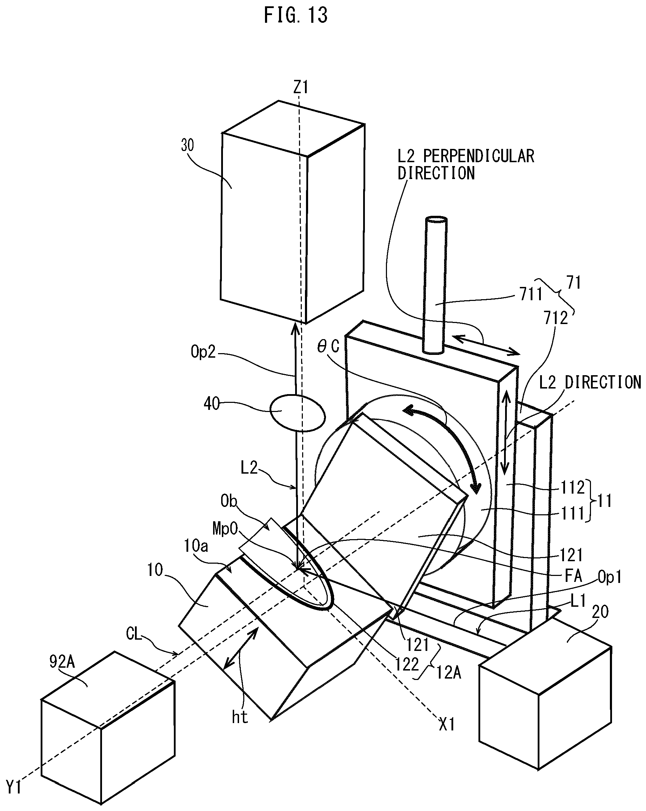

A prototype usable as an example of the device 1A was produced and evaluated. FIG. 13 is a schematic diagram illustrating a structure of the example of the device 1A.

In the example of the device 1A, a laser light source having a wavelength selected from the range of 2.5-12 µm, inclusive, is used as the light emission unit 20, and the incident angle A is set to 65 degrees. A near-infrared/mid-infrared sensor is used as the photodetector 30, and the installation angle B is set to 25 degrees. As the imaging lens 40, a lens having a focal length of f = 25 mm is used. The imaging lens 40 is installed at a position that is approximately 50 mm from both the laser-beam condensing region FA in the subject portion Mp0 and the light receiving surface 30a of the photodetector 30, and the photodetector 30 is installed at the image transfer (focus) position of the laser-beam condensing region FA. Here, the focus depth of the detector is set to a depth selected from the range of 0.5-3.5 mm, inclusive, inwards of the surface of the skin of the living body Ob, and is set to 1.5 mm in the present example.

Furthermore, as illustrated in FIG. 13, the base 10 is made from a rectangular-parallelepiped-shaped member in the example of the device 1A. A guide member 122 for regulating the position (X1A, Y1A) of the living body Ob in the in-plane direction (X1-Y1 direction) parallel to the main surface 10a is disposed on the main surface 10a of the base 10, on which the living body Ob that is the subject is placed. By installing the guide member 122 while checking the position thereof from the direction of the normal to the main surface 10a, the accuracy of the position of the living body Ob in the in-plane direction parallel to the main surface 10a is ensured.

A holding member 121 is disposed on a side surface of the base 10 to allow the base 10 to slide reversibly in the direction perpendicular to the main surface 10a. Thus, the holding member 121 has the function of changing the height ht of the living body Ob in the direction perpendicular to the main surface 10a. In the example of the device 1A, the functions of the position adjustment mechanism 12A are realized by the guide member 122 and the holding member 121.

Next, the example of the device 1A includes a rotor 111 and a stator member 112 constituting the rotation stage mechanism 11. Specifically, as illustrated in FIG. 13, further sideways from the holding member 121 disposed on a side surface of the base 10, the rotor 111 is mechanically coupled to a side surface of the holding member 121. The rotor 111 is rotatably held by the stator member 112 via a rotary shaft CL that is parallel to the axis Y1, and can rotate reversibly about the rotary shaft CL. The rotary shaft CL is disposed at a position where the rotary shaft CL intersects the subject portion Mp0 of the living body Ob placed on the main surface 10a of the base 10.

Furthermore, a lead screw 711 is inserted into the stator member 112. The lead screw 711 is provided standing upright from a base member 712 in the direction parallel to the optical path Op2 from the laser-beam condensing region FA in the subject portion Mp0 to the photodetector 30. The stator member 112 and the base 10 coupled thereto are capable of reversibly moving linearly in the direction parallel to the optical path Op2 by the lead screw 711 rotating.

Furthermore, the stator member 112 includes a linear motion mechanism (unillustrated) capable of sliding reversely relative to the base member 712 in the direction perpendicular to the optical path Op2. Due to the linear motion mechanism, the base 10 coupled to the stator member 112 can move reversibly in the direction perpendicular to the optical path Op2. As described above, in the example of the device 1A, the functions of the movable mechanism 71 are realized by the stator member 112, the lead screw 711, and the base member 712.

Next, the example of the device 1A includes the image-capturing means 92A in the direction parallel to the main surface 10a of the base 10. As illustrated in FIG. 13, the image-capturing means 92A may be disposed on the rotational axis CL intersecting the position in space corresponding to the subject portion Mp0 of the living body Ob placed on the main surface 10a of the base 10. When a height deviation in the height direction is detected based on an image from the image-capturing means 92A, the height deviation of the living body Ob is compensated for by changing the height ht of the base 10 in the direction perpendicular to the main surface 10a.

Note that, in the present example, the image-capturing means 91A, which captures an image of the living body Ob in the in-plane direction (X1-Y1 direction) parallel to the main surface 10a is omitted, and the function for regulating the position (X1A, Y1A) in the in-plane direction is realized by the guide member 122.

### (Comparative Example)

SMBG-based blood-sugar-level measurement results measured using a commercially available blood-sugar-level measurement device as a comparative example were used.

### (Test Method)

Using the back side of the index finger of an adult male subject person as the subject, blood-sugar measurement was performed at substantially fixed time intervals from noon to 9:30 PM using the example based on the device 1A and the SMBG-based comparative example. Measurement using the device 1A was performed by placing the living body Ob on the main surface of the base, and the measurement using the example and the measurement using the comparative example were performed at the same time.

### (Test Results)

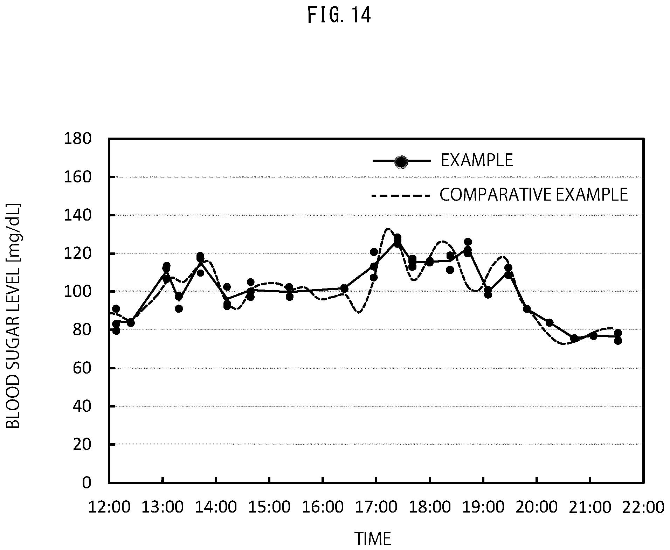

FIG. 14 is a diagram illustrating results of a substance-in-blood concentration measurement test in which the example of the device 1A and the SMBG-based comparative example were used. As illustrated in FIG. 14, in average, a measurement value obtained using the example was within a range of ±5% from a measurement value obtained using the comparative example at the same time point, and a high level of consistency could be seen between the results obtained using the example and the results obtained using the comparative example, which is a commercially available product indicating the present state of the art. Thus, it was confirmed that, according to the device 1A, accurate measurement can be stably performed by suppressing a fluctuation in measurement results caused by a difference in the manner in which a measurement-target part is placed on the device.

### <Conclusion>

As described above, the substance-in-blood concentration measurement device 1A according to Embodiment 2 may further include, in the structure according to Embodiment 1, an image-capturing means 91A and an image-capturing means 92A that each capture an image including the living body Ob, wherein a measurement controller 60A may detect an image portion corresponding to the living body Ob from an acquired image and calculate a positional deviation amount from a reference position where the image portion should be located, and the base 10 may be capable of changing the height of the main surface 10a in the perpendicular direction so as to compensate for the positional deviation amount.

According to this structure, using the image-capturing means 91A and the image-capturing means 92A, the positional relationship of the living body Ob relative to the light emission unit 20 can be adjusted easily in accordance with the shape of the individual living body Ob so that the laser beam is condensed and emitted to the blood-vessel region Mp inwards of the epidermis in the subject portion Mp0.

Furthermore, the measurement controller 60A may calculate, from an acquired image, an angular deviation amount from a reference angle in which the image portion corresponding to the living body Ob should be oriented, and the base 10 may be capable of changing the angle of the main surface 10a relative to the optical path Op1 of the laser beam L1 within a plane that is defined by the optical path Op1 of the laser beam L1 and the optical path Op2 from the subject portion to the photodetector 30 so as to compensate for the angular deviation amount calculated based on the acquired image.

According to this structure, the incident angle A and the installation angle B relative to the subject portion Mp0 can be adjusted with ease simultaneously in accordance with the shape of the individual living body Ob. Thus, a subject person can perform substance-in-blood concentration measurement more easily without being constrained by the manner in which the measurement-target part is to be placed on the device, the laser incident angle, or the photodetector installation angle.

### <<Embodiment 3>>

A substance-in-blood concentration measurement device 1B according to Embodiment 3 will be described with reference to the drawings. In the above-described examples, the back side of a finger serving as the living body Ob is measured as the subject portion Mp0 in a state in which the palm side of the finger is placed into contact with the base 10. However, measurement may be performed on a subject portion in a state in which a different part of the body, instead of a finger, is measured as the living body Ob.

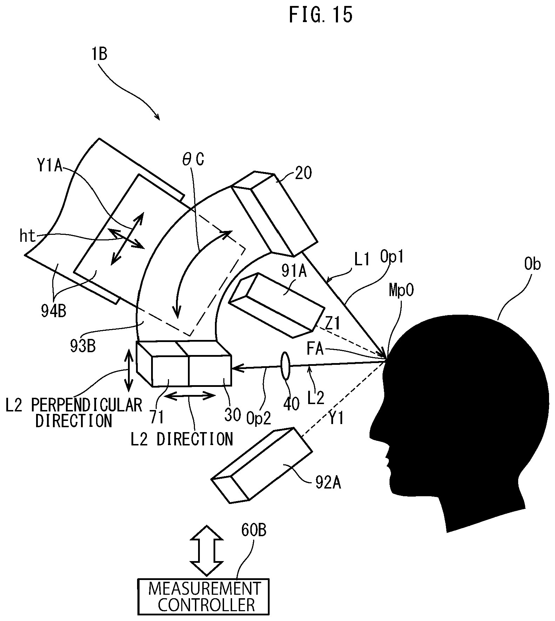

FIG. 15 is a schematic diagram illustrating a state of the substance-in-blood concentration measurement device 1B according to Embodiment 3 during measurement. The substance-in-blood concentration measurement device 1B (also referred to hereinafter as "device 1B") performs measurement by emitting a laser beam to the forehead, instead of a finger, as the living body Ob, and does not include the base 10 on which the living body Ob is placed. The device 1B performs measurement by detecting the positions of the subject portion Mp0 and the laser-beam condensing region FA using the image-capturing means 91A and 92A, and controlling the position of the laser-beam condensing position based on the detection result so that the subject portion Mp0 and the laser-beam condensing region FA are substantially aligned. According to this structure, substance-in-blood concentration measurement can be performed more easily.

Note that the device 1B according to Embodiment 3 may perform measurement on a different part of the body where the skin is disposed, other than the forehead, as the measurement-target subject portion Mp0.

As was the case in the device 1, in the device 1B, the laser beam L1 emitted from the light emission unit 20 is emitted to the subject portion Mp0 of the living body Ob, and the signal light L2 reflected by the subject portion Mp0 is received by the photodetector 30. The structures of the light emission unit 20, the photodetector 30, the imaging lens 40, and the movable mechanism 71 in the device 1B are the same as the structures of those in the device 1 in Embodiment 1 and device 1A in Embodiment 2.

### (Light-Emission-Angle Adjustment Mechanism 93B)

The device 1B includes, between the light emission unit 20 and the photodetector 30, a light-emission-angle adjustment mechanism 93B that is a structural member that holds the light emission unit 20 and holds the photodetector 30 via the movable mechanism 71. This light-emission-angle adjustment mechanism 93B has the function of regulating the positions and angles of the photodetector 30 and the light emission unit 20 so that the optical path Op1 of the laser beam L1 and the optical path Op2 of the signal light L2 intersect at a predetermined position.

The light-emission-angle adjustment mechanism 93B includes an angle changing mechanism that includes an internal motor and can reversively rotate about the rotational axis CL, which is parallel to the axis Y1. The rotational axis CL may intersect the position in a three-dimensional space where the subject portion Mp0 is present. The angles of the optical path Op1 of the laser beam L1 and the optical path Op2 of the signal light L2 relative to the surface of the skin of the living body Ob can be changed within the laser-beam incident plane by the light-emission-angle adjustment mechanism 93B rotating.

Specifically, the motor is driven based on a control signal from a measurement controller 60B, and the light-emission-angle adjustment mechanism 93B thereby actuates the angle changing mechanism. Furthermore, the light-emission-angle adjustment mechanism 93B rotates, about the rotational axis CL, the light emission unit 20 and the photodetector 30, which are held by the light-emission-angle adjustment mechanism 93B. Due to this, the incident angle A of the laser beam L1 emitted from the light emission unit 20 on the subject portion Mp0 and the installation angle B of the photodetector 30 relative to the subject portion Mp0 can be adjusted simultaneously. Thus, according to the light-emission-angle adjustment mechanism 93B, the incident angle A and the installation angle B can be respectively set to the predetermined angles θA and θB simultaneously.

### (Position Adjustment Mechanism 94B)

A position adjustment mechanism 94B is provided contiguously to the light-emission-angle adjustment mechanism 93B. The position adjustment mechanism 94B changes the position of the light-emission-angle adjustment mechanism 93B within the laser-beam incident plane. By moving the light-emission-angle adjustment mechanism 93B within the laser-beam incident plane by actuating the position adjustment mechanism 94B based on a control signal from the measurement controller 60B, the point where the optical path Op1 of the laser beam L1 with respect to the surface of the skin of the living body Ob and the optical path Op2 of the signal light L2 intersect can be moved to the position that is to become the subject portion Mp0 of the living body Ob.

Furthermore, as was the case in the device 1, the movable mechanism 71 is present between the photodetector 30 and the light-emission-angle adjustment mechanism 93B so that the position of the photodetector 30 relative to the light-emission-angle adjustment mechanism 93B can be changed. That is, by actuating the movable mechanism 71 based on a control signal from the measurement controller 60B, the photodetector 30 can be reversibly moved in a direction parallel with and in a direction perpendicular to the optical path Op2 of the signal light L2 from the the subject portion Mp0 to the photodetector 30. For example, a linear motion mechanism in which a lead screw or the like is used, or a MEMS actuator in which a piezoelectric element is used may be used as the movable mechanism 71. Furthermore, the movable mechanism 71 may move the imaging lens 40 together with the photodetector 30.

### (Image-Capturing Means 91A and 92A)

The device 1B includes an image-capturing means 91A that is disposed in the normal direction of the surface of the skin near the subject portion Mp0 of the living body Ob, and an image-capturing means 92A that is disposed in a direction parallel with the surface of the skin.

The image-capturing means 91A and the image-capturing means 92A are image-capturing means that capture images of the living body Ob. The image-capturing means 91A is disposed in the normal direction of the surface of the skin of the living body Ob so as to face the direction of the subject portion Mp0, captures a plan-view image of the surface of the skin of the living body Ob, and outputs the image data to the measurement controller 60B. Furthermore, the image-capturing means 92A is disposed sideways of the surface of the skin of the living body Ob so as to face the direction of the subject portion Mp0, captures an image of the living body Ob as seen from sideways, and outputs the image data to the measurement controller 60B.

The measurement controller 60B detects an image portion corresponding to the living body Ob from a received image, calculates a positional deviation amount from a reference position where the image portion should be located, and outputs a control signal for compensating for the positional deviation amount to the position adjustment mechanism 94B.

Specifically, for example, when a height deviation in the height direction is detected based on an image from the image-capturing means 92A, the position adjustment mechanism 94B compensates for the height deviation of the living body Ob by changing the height ht in the direction perpendicular to the surface of the skin of the living body Ob based on a control signal. Similarly, for example, when a positional deviation in the in-plane direction is detected based on an image from the image-capturing means 91A, the position adjustment mechanism 94B compensates for the positional deviation of the light emission unit 20 and the photodetector 30 in the in-plane direction by changing the position X1A, Y1A of the light-emission-angle adjustment mechanism 93B in the direction (X1-Y1 direction) parallel with the surface of the skin of the living body Ob based on a control signal.

Furthermore, in a state in which the laser beam L1 is emitted from the light emission unit 20 to the subject portion Mp0 of the living body Ob, the positions of the laser-beam condensing region FA and the subject portion Mp0 may be detected using the image-capturing means 91A and 92A, and the position adjustment mechanism 94B and/or the light-emission-angle adjustment mechanism 93B are/is driven based on the detection results to perform control of positions so that the subject portion Mp0 and the laser-beam condensing region FA are substantially aligned with one another.

According to this structure, a subject person can perform substance-in-blood concentration measurement more easily without being constrained by the manner in which the measurement-target part is to be placed on the device, the laser incident angle, or the photodetector installation angle.

### <Conclusion>

As described above, the substance-in-blood concentration measurement device 1B according to Embodiment 3 may further include, in the structure according to Embodiment 2, a light-emission-angle adjustment mechanism 93B that holds the light emission unit 20 and the photodetector 30 and that, by rotating, changes the angles of the optical path Op2 of the signal light L2 and the optical path Op1 of the laser beam L1 with respect to the surface of the skin of the living body Ob within a laser-beam incident plane.

According to this structure, the incident angle A of the laser beam L1 emitted from the light emission unit 20 on the subject portion Mp0 and the installation angle B of the photodetector 30 relative to the subject portion Mp0 can be adjusted simultaneously, and the positions and angles of the photodetector 30 and the light emission unit 20 can be regulated so that the optical path Op1 of the laser beam L1 and the optical path Op2 of the signal light L2 intersect at a predetermined position.

Furthermore, a position adjustment mechanism 94B that changes the position of the light-emission-angle adjustment mechanism 93B within the laser-beam incident plane may be further included. According to this structure, the point of intersection between the optical path Op2 of the signal light L2 and the optical path Op1 of the laser beam L1 with respect to the surface of the skin of the living body Ob can be moved to a position that is to become the subject portion Mp0 of the living body Ob.

According to this structure, a subject person can perform substance-in-blood concentration measurement more easily without being constrained by the manner in which the measurement-target part is to be placed on the device, the laser incident angle, or the photodetector installation angle.

### <<Modifications>>

Up to this point, specific structures of the present disclosure have been described taking embodiments as examples, but the present disclosure is not limited by the above embodiments in any way, except for essential characteristic constituent elements thereof. For example, an embodiment that can be obtained by applying various modifications to the embodiments, and an embodiment that can be realized by combining constituent elements and functions in the embodiments are also included in the present disclosure, the scope of the present invention being defined by the appended claims.

(1) In the embodiments, an embodiment has been described taking glucose as an example of the measurement-target substance (first substance in blood) to be detected by the substance-in-blood concentration measurement device. However, components in blood that can be detected by the substance-in-blood concentration measurement device according to the present disclosure is not limited to the above, and the device can be widely used for other detection targets by changing the wavelength of the laser beam L1 emitted by the light emission unit 20 according to the type of component in blood.
   For example, the wavelength of the laser beam L1 emitted by the light emission unit 20 may be 8.23 ± 0.05 µm (8.18-8.28 µm, inclusive), and the component in blood may be lactic acid. Alternatively, the wavelength may be within the range of -0.05 µm to +0.05 µm, inclusive, from 5.77 µm, 6.87 µm, 7.27 µm, 8.87 µm, or 9.55 µm. The inventor, etc., have confirmed through experimentation that lactic-acid concentration measurement values obtained by the photodetector when the wavelength of light emitted by the light emission unit 20 is set to 8.23 µm are generally correlated with lactic-acid-value measurement results obtained by SMBG.
(2) In the embodiments, an embodiment has been described taking hemoglobin as an example of the reference substance (second substance in blood) detected in the reference measurement. However, the reference substance according to the present disclosure is not limited to the above, and application to other reference substances is possible by changing the wavelength of the laser beam L1 emitted by the light emission unit 20 according to the type of component in blood to be used as the reference substance.
(3) In the embodiments, the wavelength of the oscillated laser beam L1 can be switched and adjusted by adjusting the type and matching conditions of the nonlinear optical crystal 223 in the OPO 22.

However, a device that can measure a plurality of types of components in blood may be realized by adopting, in the light emission unit 20, a device structure such that a plurality of OPOs 22 can be selectively used, and laser beams L1 of a plurality of wavelengths can be selectively emitted. Light emitted from the light source 21 may be switchingly made to enter the plurality of OPOs emitting light of different wavelengths so that light of different wavelengths is selectively emitted from the OPOs, and the reference measurement and the main measurement of the measurement target can be selectively performed using the different wavelengths.

Alternatively, a plurality of light emission units 20 emitting light of different wavelengths may be used, and the light from two light emission units 20 may be selectively emitted as the laser beam L1 using an optical coupler, a mirror, etc. Differing from a structure in which optical systems having different optical path width, thickness, etc., are used depending on the wavelengths of light, this enables the same optical system formed from the condenser lens 50, the base 10, the imaging lens 40, and the photodetector 30 capable of detecting mid-infrared light to be used as an optical system for a plurality of components in blood, for example.

(4) In the embodiments, in regard to the light emission unit 20, the first laser beam for target measurement and the second laser beam for reference measurement have different wavelengths. However, as long as the first laser beam for target measurement and the second laser beam for reference measurement are respectively absorbed by the measurement-target substance and the reference substance, an emission condition other than wavelength may be varied between the first laser beam and the second laser beam. For example, the intensity of light emitted from the emission unit may be varied between the first laser beam and the second laser beam.

(5) In the embodiments, the measurement-target substance and the reference substance are different substances in blood. However, the reference measurement may be performed using the same substance as the measurement-target substance.

(6) In the embodiments, an embodiment of a substance-in-blood concentration measurement device has been described taking as an example an optical system including the imaging lens 40 between the subject portion Mp0 and the photodetector 30. However, as long as the substance-in-blood concentration measurement device according to the present disclosure forms a focused image on the photodetector 30 from the signal light L2 reflected from the blood-vessel region Mp of the living body Ob, the structure of the light-receiving-side optical system may be changed, as appropriate. For example, a structure in which a plurality of lenses are used or a structure in which a mirror is disposed in the middle of the optical path may be adopted.

(7) The order in which steps in processing are executed in the embodiment is an example, and an order other than the order above may be adopted. Furthermore, some of the steps in processing may be executed concurrently with (in parallel with) other steps.

Furthermore, at least some of the functions in the embodiments and the modifications thereof may be combined with one another.

### <<Supplement>>

The embodiments described above are preferred specific examples. Numerical values, shapes, materials, constituent elements, arrangements, positions, and connections of constituent elements, processes, order of processes, and the like described above are illustrative examples of embodiments, and are not intended to limit the present invention.

Furthermore, the order according to which the above-described method is executed is an example, and an order other than the order above may be adopted. Furthermore, parts of the above-described method may be performed concurrently with (in parallel with) other methods.

Furthermore, in order to facilitate understanding, constituent elements in the drawings mentioned in the embodiments are not necessarily drawn to scale.

Furthermore, all numbers used above are examples.

### [Industrial Applicability]

The substance-in-blood concentration measurement device, substance-in-blood concentration measurement method, and program according to aspects of the present disclosure can be widely used in medical devices for the daily measurement of states of substances in blood, such as the blood sugar level and blood lipid level, in the prevention and treatment of lifestyle-related diseases.

### [Reference Signs List]

- 1, 1A, 1B: Substance-in-blood concentration measurement device
- 10: Base
- 11: Rotation stage mechanism
- 12: Height adjustment mechanism
- 12A: Position adjustment mechanism
- 20: Light emission unit
- 21: Light source
- 22: Optical parametric oscillator
- 221: Incident-side semi-transmissive mirror
- 222: Output-side semi-transmissive mirror
- 223: Nonlinear optical crystal
- 30: Photodetector
- 30a: Screen (light receiving surface)
- 40: Imaging lens (first lens)
- 50: Condenser lens (second lens)
- 60, 60A, 60B: Measurement controller
- 70: Light detection unit
- 71: Movable mechanism
- 711: First linear transportation mechanism
- 712: Second linear transportation mechanism
- 80: Diaphragm
- 80a: Opening
- 91A: Image-capturing means
- 92A: Image-capturing means
- 93B: Light-emission-angle adjustment mechanism
- 94B: Position adjustment mechanism

## Claims

1. A substance-in-blood concentration measurement device (1; 1A) for measuring a concentration of a substance in blood included in the blood in a subject portion (Mp0) of a living body (Ob), the substance-in-blood concentration measurement device (1; 1A) comprising:
a base (10) having a main surface (10a) on which the living body (Ob) can be placed;
a light emission unit (20) that is disposed on the main surface-side (10a) of the base (10) and configured to condense and emit a laser beam (L1) to a specific region in the subject portion (Mp0), the specific region being present in skin located on a side of the living body (Ob) which is the reverse side from a skin surface on a side of the living body (Ob) facing the main surface (10a);
a photodetector (30) that is disposed on the main surface-side (10a) of the base (10) and configured to receive signal light (L2) that is reflected light based on the laser beam (L1), and detect an intensity of the signal light (L2), the signal light (L2) having lower light intensity than the laser beam (L1) in some wavelengths;
an imaging lens (40) that is disposed in a position between the subject portion (Mp0) and the photodetector (30) where a focused image (Im2, Im21) of the signal light (L2) from a laser-beam condensing region (FA) in the subject portion (Mp0) can be formed on the photodetector (30); and
a measurement controller (60; 60A) that is configured to measure a concentration of the substance in blood in the laser-beam condensing region (FA) based on the intensity of the signal light (L2), wherein the configuration is such that:
a first angle (*θ*A) formed by a normal to the surface of the skin of the subject portion (Mp0) and an optical path (Op1) of the laser beam (L1) is different from a second angle (*θ*B) formed by the normal and an optical path (Op2) of the signal light (L2) from the laser-beam condensing region (FA) to the photodetector (30),
the position of the living body (Ob) relative to the light emission unit (20) is set so that the laser-beam condensing region (FA) and a blood-vessel region (Mp) inwards of the epidermis in the subject portion (Mp0) overlap, and
the position of the photodetector (30) relative to the living body (Ob) is set so that an image (Im11) of the signal light (L2) from the blood-vessel region (Mp) overlapping the laser-beam condensing region (FA) is transferred by the imaging lens (40) to form a focused image (Im21) on a light receiving surface (30a) of the photodetector (30).

2. The substance-in-blood concentration measurement device (1; 1A) according to claim 1, wherein
the base (10) is capable of adjusting the position of the living body (Ob) relative to the light emission unit (20) by changing the height (ht) of the main surface (10a) in a direction perpendicular to the main surface (10a).

3. The substance-in-blood concentration measurement device (1; 1A) according to claim 1, wherein
the position of the photodetector (30) relative to the living body (Ob) in a direction intersecting the optical path (Op2) of the signal light (L2) is capable of being adjusted by changing the position of the photodetector (30) in said direction.

4. The substance-in-blood concentration measurement device (1; 1A) according to claim 1, wherein
the base (10) is capable of adjusting the first angle (*θ*A) and the second angle (*θ*B) simultaneously by changing the angle (*θ*C) of the main surface (10a) relative to the optical path (Op1) of the laser beam (L1) within a plane that is defined by the optical path (Op1) of the laser beam (L1) and the optical path (Op2) from the subject portion (Mp0) to the photodetector (30).

5. The substance-in-blood concentration measurement device (1; 1A) according to claim 1, wherein
the position of the photodetector (30) relative to the living body (Ob) in a direction along the optical path (Op2) of the signal light (L2) is capable of being adjusted by changing the position of the photodetector (30) in said direction, and
the position of the photodetector (30) is adjusted based on the concentration of the substance in blood so that the image (Im11) of the signal light (L2) from the blood-vessel region (Mp) is transferred by the imaging lens (40) to form a focused image (Im21) on the light receiving surface (30a) of the photodetector (30).

6. The substance-in-blood concentration measurement device (1; 1A) according to any one of claims 1 to 5, wherein
the light emission unit (20) is capable of selectively emitting: a first laser beam for a target measurement that is absorbed by a first substance in blood that is a measurement-target substance; and a second laser beam for a reference measurement that is absorbed by a second substance in blood that is a reference substance, and
an absorption rate at which the second laser beam is absorbed by the second substance in blood in the reference measurement is higher than an absorption rate at which the first laser beam is absorbed by the first substance in blood in the target measurement.

7. The substance-in-blood concentration measurement device (1; 1A) according to claim 6, wherein
a concentration of the reference substance in blood is more stable than a concentration of the measurement-target substance in blood.

8. The substance-in-blood concentration measurement device (1; 1A) according to claim 6, wherein
the measurement controller (60; 60A) is capable of measuring, based on emission of the second laser beam, a concentration of the second substance in blood in a state in which the specific region is included in the blood-vessel region (Mp) in the subject portion (Mp0), and
is capable of measuring, based on emission of the first laser beam, a concentration of the first substance in blood in the specific region as a concentration of the first substance in blood in the measurement-target portion.

9. The substance-in-blood concentration measurement device (1; 1A) according to claim 1 further comprising
a condenser lens (50) that is positioned between the light emission unit (20) and the subject portion (Mp0) in the optical path (Op1) of the laser beam (L1), and that condenses the laser beam (L1) to the laser-beam condensing region (FA).

10. The substance-in-blood concentration measurement device (1; 1A) according to claim 9, wherein
within a section from the base (10) to the photodetector (30) in the optical path (Op2) from the subject portion (Mp0) to the photodetector (30), the signal light (L2) propagates through a space except within a section in which the signal light (L2) passes through the imaging lens (40), and
within a section from the light emission unit (20) to the base (10) in the optical path (Op1) from the light emission unit (20) to the subject portion (Mp0), the laser beam (L1) propagates through a space except within a section in which the laser beam (L1) passes through the condenser lens (50).

11. The substance-in-blood concentration measurement device (1; 1A) according to any one of claims 1 to 5 or claim 9 further comprising
an image-capturing means (91A; 92A) that is configured to capture an image including the living body (Ob), wherein
the measurement controller (60; 60A) is configured to detect an image portion corresponding to the living body (Ob) from the acquired image and to calculate a positional deviation amount from a reference position where the image portion should be located, and
the base (10) is capable of changing the height (ht) of the main surface (10a) in a perpendicular direction so as to compensate for the positional deviation amount.

12. The substance-in-blood concentration measurement device (1; 1A) according to any one of claims 1 to 5 or claim 9 further comprising
an image-capturing means (91A; 92A) that is configured to capture an image including the living body (Ob), wherein
the measurement controller (60; 60A) is configured to detect an image portion corresponding to the living body (Ob) from the acquired image and to calculate an angular deviation amount from a reference angle in which the image portion should be oriented, and
the base (10) is capable of changing the angle (*θ*C) of the main surface (10a) with respect to the optical path (Op1) of the laser beam (L1) so as to compensate for the angular deviation amount.

13. The substance-in-blood concentration measurement device (1; 1A) according to claim 1 further comprising
a light-emission-angle adjustment mechanism (93B) that holds the light emission unit (20) and the photodetector (30) and that is configured to, by rotating, change the angle (*θ*B) of the optical path (Op2) of the signal light (L2) and the angle (*θ*A) of the optical path (Op1) of the laser beam (L1) with respect to the surface of the skin of the living body (Ob) within a laser-beam incident plane.

14. The substance-in-blood concentration measurement device (1; 1A) according to claim 13 further comprising
a position adjustment mechanism (12A; 94B) that is configured to change the position of the light-emission-angle adjustment mechanism (93B) within the laser-beam incident plane.

15. A substance-in-blood concentration measurement method for measuring a concentration of a substance in blood included in the blood in a subject portion (Mp0) of a living body (Ob), the substance-in-blood concentration measurement method comprising:
placing the living body (Ob) on a main surface (10a) of a base (10), and condensing and emitting a laser beam (L1) for a target measurement to a specific region in the subject portion (Mp0) by means of a light emission unit (20) which is disposed on the main-surface side (10a) of the base (10),
the specific region being present in skin located on a side of the living body (Ob) which is the reverse side from a skin surface on a side of the living body (Ob) facing the main surface (10a), and the laser beam (L1) for the target measurement being absorbed by a substance in blood that is a measurement-target substance;
using an imaging lens (40) that is positioned between the subject portion (Mp0) and a photodetector (30), which is disposed on the main surface-side (10a) of the base (10), to form a focused image (Im2, Im21) of signal light (L2) on the photodetector (30), the signal light (L2) being reflected light of the laser beam (L1), being emitted from a laser-beam condensing region (FA) in the subject portion (Mp0), and having lower light intensity than the laser beam (L1) in some wavelengths; and
receiving the signal light (L2) by means of the photodetector (30) and measuring a concentration of the substance in blood in the specific region based on the intensity of the received signal light (L2), wherein
a first angle (*θ*A) formed by a normal to the surface of the skin of the subject portion (Mp0) and an optical path (Op1) of the laser beam (L1) is different from a second angle (*θ*B) formed by the normal and an optical path (Op2) of the signal light (L2) from the specific region to the photodetector (30),
the position of the living body (Ob) relative to the light emission (20) unit is set so that the laser-beam condensing region (FA) and a blood-vessel region (Mp) inwards of the epidermis in the subject portion (Mp0) overlap, and
the position of the photodetector (30) relative to the living body (Ob) is set so that an image (Im11) of the signal light (L2) from the blood-vessel region (Mp) overlapping the laser-beam condensing region (FA) is transferred by the imaging lens (40) to form a focused image (Im21) on a light receiving surface (30a) of the photodetector (30).

16. The substance-in-blood concentration measurement method according to claim 15 further comprising
prior to the measuring, adjusting the position of the living body (Ob) relative to the light emission unit (20) by changing the height (ht) of the main surface (10a) of the base (10) in a direction perpendicular to the main surface (10a).

17. The substance-in-blood concentration measurement method according to claim 15 further comprising
prior to the measuring, adjusting the position of the photodetector (30) relative to the living body (Ob) in a direction intersecting the optical path (Op2) of the signal light (L2) by changing the position of the photodetector (30) in said direction.

18. The substance-in-blood concentration measurement method according to claim 15 further comprising
prior to the target measurement, adjusting the first angle (*θ*A) and the second angle (*θ*B) simultaneously based on the concentration of the substance in blood by performing a reference measurement in which the concentration of the substance in blood in the specific region is measured while changing the angle (*θ*C) of the main surface (10a) within a plane that is defined by the optical path (Op1) of the laser beam (L1) and the optical path (Op2) from the subject portion (Mp0) to the photodetector (30).

19. The substance-in-blood concentration measurement method according to claim 15 further comprising
prior to the target measurement, adjusting the position of the photodetector (30) relative to the living body (Ob) in a direction along the optical path (Op2) of the signal light (L2) by performing a reference measurement in which the concentration of the substance in blood is measured while changing the position of the photodetector (30) along the optical path (Op2) of the signal light (L2), wherein
in the adjusting, the position of the photodetector (30) is adjusted based on the concentration of the substance in blood so that an image (Im11) of the signal light (L2) from the blood-vessel region (Mp) is transferred by the imaging lens (40) to form a focused image (Im21) on the light receiving surface (30a) of the photodetector (30).

20. The substance-in-blood concentration measurement method according to any one of claims 15 to 19, wherein
when the laser beam, the substance in blood, and the concentration of the substance in blood are respectively referred to as a first laser beam, a first substance in blood, and a concentration of the first substance in blood,
in the reference measurement:
a second laser beam for the reference measurement that is absorbed by a second substance in blood that is a reference substance is emitted from the light emission unit (20) to the irradiated region;
using the imaging lens (40), a focused image (Im21) is formed on the photodetector (30) from signal light (L2) of the second laser beam reflected from the specific region;
the signal light (L2) of the second laser beam is received by means of the photodetector (30), and a concentration of the second substance in blood based on the signal light (L2) is measured as a concentration of the second substance in blood in the measurement-target portion; and
an absorption rate at which the second laser beam is absorbed by the second substance in blood is higher than an absorption rate at which the first laser beam is absorbed by the first substance in blood in the target measurement.

21. The substance-in-blood concentration measurement method according to claim 20, wherein the concentration of the reference substance in blood is more stable than the concentration of the measurement-target substance in blood.

22. The substance-in-blood concentration measurement method according to claim 20 further comprising:
measuring, based on emission of the second laser beam, the concentration of the second substance in blood in a state in which the specific region is included in the blood-vessel region (Mp) in the subject portion (Mp0), and
measuring, based on emission of the first laser beam, the concentration of the first substance in blood in the specific region as the concentration of the first substance in blood in the measurement-target portion.

## Patentansprüche

1. Vorrichtung (1; 1A) für Messung einer Konzentration von Substanzen in Blut, mit der eine Konzentration einer Substanz in Blut gemessen wird, die in dem Blut eines Zielabschnitts (Mp0) eines lebenden Körpers (Ob) enthalten ist, wobei die Vorrichtung (1; 1A) für Messung einer Konzentration von Substanzen in Blut umfasst:
eine Auflage (10), die eine Hauptfläche (10a) aufweist, auf die der lebende Körper (Ob) aufgelegt werden kann;
eine Lichtemissionseinheit (20), die an der Seite der Hauptfläche (10a) der Auflage (10) angeordnet und so ausgeführt ist, dass sie einen Laserstrahl (L1) bündelt und auf einen bestimmten Bereich in dem Zielabschnitt (Mp0) emittiert, wobei der bestimmte Bereich in Haut liegt, die sich an einer Seite des lebenden Körpers (Ob) befindet, die die Rückseite gegenüber einer Hautoberfläche an einer Seite des lebenden Körpers (Ob) ist, die der Hauptfläche (10a) zugewandt ist;
einen Fotodetektor (30), der an der Seite der Hauptfläche (10a) der Auflage (10) angeordnet und so ausgeführt ist, dass sie Signallicht (L2) empfängt, das auf dem Laserstrahl (L1) basierendes reflektiertes Licht ist, und eine Stärke des Signallichts (L2) erfasst, wobei das Signallicht (L2) bei einigen Wellenlängen eine geringere Lichtstärke hat als der Laserstrahl (L1);
eine Abbildungslinse (40), die an einer Position zwischen dem Zielabschnitt (Mp0) und dem Fotodetektor (30) angeordnet ist, wobei ein fokussiertes Bild (lm2, lm21) des Signallichts (L2) von einem Laserstrahl-Bündelungsbereich (FA) in dem Zielabschnitt (Mp0) auf dem Fotodetektor (30) erzeugt werden kann; und
eine Messungs-Steuerung (60; 60A), die so ausgeführt ist, dass sie eine Konzentration der Substanz in Blut in dem Laserstrahl-Bündelungsbereich (FA) auf Basis der Stärke des Signallichts (L2) misst, wobei die Konfiguration derart ist, dass:
ein erster Winkel (θA), der durch eine Senkrechte zu der Hautoberfläche des Zielabschnitts (Mp0) und einen Strahlengang (Op1) des Laserstrahls (L1) gebildet wird, sich von einem zweiten Winkel (θB) unterscheidet, der durch die Senkrechte und einen Strahlengang (Op2) des Signallichts (L2) von dem Laserstrahl-Bündelungsbereich (FA) zu dem Fotodetektor (30) gebildet wird,
die Position des lebenden Körpers (Ob) relativ zu der Lichtemissionseinheit (20) so eingestellt ist, dass der Laserstrahl-Fokussierbereich (FA) und ein Blutgefäßbereich (Mp) innerhalb der Epidermis in dem Zielabschnitt (Mp0) einander überlagern, und
die Position des Fotodetektors (30) relativ zu dem lebenden Körper (Ob) so eingestellt ist, dass ein Bild (Im11) des Signallichts (L2) aus dem Blutgefäßbereich (Mp), der den Laserstrahl-Bündelungsbereich (FA) überlagert, durch die Abbildungslinse (40) so übertragen wird, dass ein fokussiertes Bild (Im21) auf einer Lichtempfangsfläche (30a) des Fotodetektors (30) erzeugt wird.

2. Vorrichtung (1; 1A) für Messung einer Konzentration von Substanzen in Blut nach Anspruch 1, wobei
die Auflage (10) in der Lage ist, die Position des lebenden Körpers (Ob) relativ zu der Lichtemissionseinheit durch Ändern der Höhe (ht) der Hauptfläche (10a) in einer Richtung senkrecht zu der Hauptfläche (10a) zu verstellen.

3. Vorrichtung (1; 1A) für Messung einer Konzentration von Substanzen in Blut nach Anspruch 1, wobei
die Position des Fotodetektors (30) relativ zu dem lebenden Körper (Ob) in einer Richtung, die den Strahlengang (Op2) des Signallichts (L2) schneidet, durch Ändern der Position des Fotodetektors (30) in der Richtung verstellt werden kann.

4. Vorrichtung (1; 1A) für Messung einer Konzentration von Substanzen in Blut nach Anspruch 1, wobei
der Sockel (10) in der Lage ist, den ersten Winkel (θA) und den zweiten Winkel θB) simultan zu verstellen, indem der Winkel (θC) der Hauptfläche (10a) relativ zu dem Strahlengang (Op1) des Laserstrahls (L1) innerhalb einer Ebene geändert wird, die durch den Strahlengang (Op1) des Laserstrahls (L1) und den Strahlengang (Op2) von dem Zielabschnitt (MpO) zu dem Fotodetektor (30) geändert wird.

5. Vorrichtung (1; 1A) für Messung einer Konzentration von Substanzen in Blut nach Anspruch 1, wobei
die Position des Fotodetektors (30) relativ zu dem lebenden Körper (Ob) in einer Richtung entlang des Strahlengangs (Op2) des Signallichts (L2) durch Ändern der Position des Fotodetektors (30) in der Richtung verstellt werden kann, und
die Position des Fotodetektors (30) auf Basis der Konzentration der Substanz in Blut so verstellt wird, dass das Bild (lm11) des Signallichts (L2) aus dem Blutgefäßbereich (Mp) durch die Abbildungslinse (40) so übertragen wird, dass ein fokussiertes Bild (lm21) auf der Lichtempfangsfläche (30a) des Fotodetektors (30) erzeugt wird.

6. Vorrichtung (1; 1A) für Messung einer Konzentration von Substanzen in Blut nach einem der Ansprüche 1 bis 5, wobei
die Lichtemissionseinheit (20) in der Lage ist, selektiv einen ersten Laserstrahl für eine Zielmessung, der durch eine erste Substanz in Blut absorbiert wird, die eine Messungsziel-Substanz ist; und einen zweiten Laserstrahl für eine Referenzmessung zu emittieren, der durch eine zweite Substanz in Blut absorbiert wird, die eine Referenz-Substanz ist, und
eine Absorptionsrate, mit der bei der Referenzmessung der zweite Laserstrahl durch die zweite Substanz in Blut absorbiert wird, höher ist als eine Absorptionsrate, mit der der erste Laserstrahl bei der Zielmessung durch die erste Substanz in Blut absorbiert wird.

7. Vorrichtung (1; 1A) für Messung einer Konzentration von Substanzen in Blut nach Anspruch 6, wobei
eine Konzentration der Referenz-Substanz in Blut stabiler ist als eine Konzentration der Messungsziel-Substanz in Blut.

8. Vorrichtung (1; 1A) für Messung einer Konzentration von Substanzen in Blut nach Anspruch 6, wobei
die Messungs-Steuerung (60; 60A) in der Lage ist, auf Basis von Emission des zweiten Laserstrahls eine Konzentration der zweiten Substanz in Blut in einem Zustand zu messen, in dem der bestimmte Bereich in dem Blutgefäßbereich (Mp) in dem Zielabschnitt (Mp0) enthalten ist, und
in der Lage ist, auf Basis von Emission des ersten Laserstrahls eine Konzentration der ersten Substanz in Blut in dem bestimmten Bereich als eine Konzentration der ersten Substanz in Blut in dem Messungsziel-Abschnitt zu messen.

9. Vorrichtung (1; 1A) für Messung einer Konzentration von Substanzen in Blut nach Anspruch 1, die des Weiteren umfasst:
eine Bündelungslinse (50), die zwischen der Lichtemissionseinheit (20) und dem Zielabschnitt (Mp0) in dem Strahlengang (Op1) des Laserstrahls (L1) positioniert ist und den Laserstrahl (L1) auf den Laserstrahl-Bündelungsbereich (FA) bündelt.

10. Vorrichtung (1; 1A) für Messung einer Konzentration von Substanzen in Blut nach Anspruch 9, wobei
innerhalb eines Teilabschnitts von der Auflage (10) zu dem Fotodetektor (30) in dem Strahlengang (Op2) von dem Zielabschnitt (Mp0) zu dem Fotodetektor (30) sich das Signallicht (L2) außer innerhalb eines Teilabschnitts, in dem das Signallicht (L2) durch die Abbildungslinse (40) hindurchtritt, durch einen Raum hindurch ausbreitet, und
innerhalb eines Teilabschnitts von der Lichtemissionseinheit (20) zu der Auflage (30) in dem Strahlengang (Op1) von der Lichtemissionseinheit (20) zu dem Zielabschnitt (Mp0) sich der Laserstrahl (L1) außer innerhalb eines Teilabschnitts, in dem der Laserstrahl (L1) durch die Bündelungslinse (50) hindurchtritt, durch einen Raum hindurch ausbreitet.

11. Vorrichtung (1; 1A) für Messung einer Konzentration von Substanzen in Blut nach einem der Ansprüche 1 bis 5 oder Anspruch 9, die des Weiteren umfasst:
eine Bildaufnahmeeinrichtung (91A, 92A), die zum Aufnehmen eines Bildes ausgeführt ist, das den lebenden Körper (Ob) einschließt, wobei
die Messungs-Steuerung (60; 60A) so ausgeführt ist, dass sie einen Bildabschnitt, der dem lebenden Körper (Ob) entspricht, aus dem erfassten Bild ermittelt und ein Positions-Abweichungsmaß von der Referenzposition berechnet, an der der Bildabschnitt sich befinden sollte, und
die Auflage (10) in der Lage ist, die Höhe (ht) der Hauptfläche (10a) in einer senkrechten Richtung so zu ändern, dass das Positions-Abweichungsmaß kompensiert wird.

12. Vorrichtung (1; 1A) für Messung einer Konzentration von Substanzen in Blut nach einem der Ansprüche 1 bis 5 oder Anspruch 9, die des Weiteren umfasst:
eine Bildaufnahmeeinrichtung (91A, 92A), die zum Aufnehmen eines Bildes ausgeführt ist, das den lebenden Körper (Ob) einschließt, wobei
die Messungs-Steuerung (60; 60A) so ausgeführt ist, dass sie einen Bildabschnitt, der dem lebenden Körper (Ob) entspricht, aus dem erfassten Bild ermittelt und ein Winkel-Abweichungsmaß von einem Referenzwinkel berechnet, in dem der Bildabschnitt ausgerichtet sein sollte, und
die Auflage (10) in der Lage ist, den Winkel (θC) der Hauptfläche (10a) in Bezug auf den Strahlengang (Op1) des Laserstrahls (L1) so zu ändern, dass das Winkel-Abweichungsmaß kompensiert wird.

13. Vorrichtung (1; 1A) für Messung einer Konzentration von Substanzen in Blut nach Anspruch 1, die des Weiteren umfasst:
einen Lichtemissionswinkel-Verstellmechanismus (93B), der die Lichtemissionseinheit (20) und den Fotodetektor (30) aufnimmt, und der so ausgeführt ist, dass er durch Drehen den Winkel (θB) des Strahlengangs (Op2) des Signallichts (L2) und den Winkel (θA) des Strahlengangs (Op1) des Laserstrahls (L1) in Bezug auf die Hautoberfläche des lebenden Körpers (Ob) innerhalb einer Laserstrahl-Auftreffebene ändert.

14. Vorrichtung (1; 1A) für Messung einer Konzentration von Substanzen in Blut nach Anspruch 13, die des Weiteren umfasst:
einen Positions-Verstellmechanismus (12A; 94B), der so ausgeführt ist, dass er die Position des Lichtemissionswinkel-Verstellmechanismus (93B) innerhalb der Laserstrahl-Auftreffebene ändert.

15. Verfahren zum Messen einer Konzentration von Substanzen in Blut, mit dem eine Konzentration einer Substanz in Blut gemessen wird, die in dem Blut eines Zielabschnitts (Mp0) eines lebenden Körpers (Ob) enthalten ist, wobei das Verfahren zum Messen einer Konzentration von Substanzen in Blut umfasst:
Auflegen des lebenden Körpers (Ob) auf eine Hauptfläche (10a) einer Auflage (10), und Bündeln sowie Emittieren eines Laserstrahls (L1) für eine Zielmessung auf einen bestimmten Bereich in dem Zielabschnitt (Mp0) mittels einer Lichtemissionseinheit (20), die an der Hauptflächen-Seite (10a) der Auflage (10) angeordnet ist, wobei der bestimmte Bereich in Haut liegt, die sich an einer Seite des lebenden Körpers (Ob) befindet, die die Rückseite gegenüber einer Hautoberfläche an einer Seite des lebenden Körpers (Ob) ist, die der Hauptfläche (10a) zugewandt ist, und der Laserstrahl (L1) für die Zielmessung durch eine Substanz in Blut absorbiert wird, die eine Messungsziel-Substanz ist;
Einsetzen einer Abbildungslinse (40), die zwischen dem Zielabschnitt (Mp 0) und einem Fotodetektor (30) positioniert ist, der an der Hauptflächen-Seite (10a) der Auflage (10) zum Erzeugen eines fokussierten Bildes (lm2, lm21) von Signallicht (L) auf dem Fotodetektor (30) angeordnet ist, wobei das Signallicht reflektiertes Licht des Laserstrahls (L1) ist, das von einem Laserstrahl-Bündelungsbereich (FA) in dem Zielabschnitt (Mp 0) emittiert wird und bei einigen Wellenlängen eine niedrigere Lichtstärke hat als der Laserstrahl (L1); sowie
Empfangen des Signallichts (L2) mittels des Fotodetektors (30) und Messen einer Konzentration der Substanz in Blut in dem bestimmten Bereich auf Basis der Stärke des empfangenen Signallichts (L2), wobei
ein erster Winkel (θA), der durch eine Senkrechte zu der Hautoberfläche des Objektbereiches (Mp0) und einen Strahlengang (Op1) des Laserstrahls (L1) gebildet wird, sich von einem zweiten Winkel (θB) unterscheidet, der durch die Senkrechte und einen Strahlengang (Op2) des Signallichts (L2) von dem bestimmten Bereich bis zu dem Fotodetektor (30) gebildet wird,
die Position des lebenden Körpers (Ob) relativ zu der Lichtemissionseinheit (20) so eingestellt ist, dass der Laserstrahl-Bündelungsbereich (FA) und ein Blutgefäßbereich (Mp) innerhalb der Epidermis in dem Zielabschnitt (Mp0) einander überlagern, und
die Position des Fotodetektors (30) relativ zu dem lebenden Körper (Ob) so festgelegt wird, dass ein Bild (Im11) des Signallichts (L2) aus dem Blutgefäßbereich (Mp), der den Laserstrahl-Bündelungsbereich (FA) überlagert, durch die Abbildungslinse (40) so übertragen wird, dass ein fokussiertes Bild (Im21) auf einer Lichtempfangsfläche (30a) des Fotodetektors (30) erzeugt wird.

16. Verfahren für Messung einer Konzentration von Substanzen in Blut nach Anspruch 15, das des Weiteren umfasst:
Verstellen der Position des lebenden Körpers (Ob) relativ zu der Lichtemissionseinheit (20) durch Ändern der Höhe (ht) der Hauptfläche (10a) der Auflage (10) in einer Richtung senkrecht zu der Hauptfläche (10a) vor dem Messen.

17. Verfahren für Messung einer Konzentration von Substanzen in Blut nach Anspruch 15, das des Weiteren umfasst:
Verstellen der Position des Fotodetektors (30) relativ zu dem lebenden Körper (Ob) in einer Richtung, die den Strahlengang (Op2) des Signallichts (L2) schneidet, durch Ändern der Position des Fotodetektors (30) in der Richtung vor dem Messen.

18. Verfahren für Messung einer Konzentration von Substanzen in Blut nach Anspruch 15, das des Weiteren umfasst:
vor der Zielmessung gleichzeitiges Verstellen des ersten Winkels (θA) und des zweiten Winkels (θB) basierend auf der Konzentration der Substanz in Blut durch Durchführen einer Referenzmessung, bei der die Konzentration der Substanz in Blut in dem bestimmten Bereich gemessen wird, während der Winkel (θC) der Hauptfläche (10a) innerhalb einer Ebene geändert wird, die durch den Strahlengang (Op1) des Laserstrahls (L1) und den Strahlengang (Op2) von dem Zielabschnitt (Mp0) zu dem Fotodetektor (30) definiert ist.

19. Verfahren für Messung einer Konzentration von Substanzen in Blut nach Anspruch 15, das des Weiteren umfasst:
vor der Zielmessung Verstellen der Position des Fotodetektors (30) relativ zu dem lebenden Körper (Ob) in einer Richtung entlang des Strahlengangs (Op2) des Signallichts (L2) durch Durchführen einer Referenzmessung, bei der die Konzentration der Substanz in Blut gemessen wird, während die Position des Fotodetektors (30) entlang des Strahlengangs (Op2) des Signallichts (L2) geändert wird, wobei
bei dem Verstellen die Position des Fotodetektors (30) auf Basis der Konzentration der Substanz in Blut so verstellt wird, dass ein Bild (lm11) des Signallichts (L2) aus dem Blutgefäßbereich (Mp) durch die Abbildungslinse (40) so übertragen wird, dass ein fokussiertes Bild (lm21) auf der Lichtempfangsfläche (30a) des Fotodetektors (30) erzeugt wird.

20. Verfahren für Messung einer Konzentration von Substanzen in Blut nach einem der Ansprüche 15 bis 19, wobei
wenn der Laserstrahl, die Substanz in Blut und die Konzentration der Substanz in Blut jeweils als ein erster Laserstrahl, eine erste Substanz in Blut bzw. eine Konzentration der ersten Substanz in Blut bezeichnet werden,
bei der Referenzmessung:
ein zweiter Laserstrahl für die Referenzmessung, der durch eine zweite Substanz in Blut absorbiert wird, die eine Referenzsubstanz ist, von der Lichtemissionseinheit (20) in den bestrahlten Bereich emittiert wird;
unter Verwendung der Abbildungslinse (40) ein fokussiertes Bild (lm21) auf dem Fotodetektor (30) aus Signallicht (L2) des zweiten Laserstrahls erzeugt wird, das von dem bestimmten Bereich reflektiert wird;
das Signallicht (L2) des zweiten Laserstrahls mittels des Fotodetektors (30) empfangen wird und eine Konzentration der zweiten Substanz in Blut auf Basis des Signallichts (L2) als eine Konzentration der zweiten Substanz in Blut in dem Messungszielabschnitt gemessen wird; und
eine Absorptionsrate, mit der der zweite Laserstrahl durch die zweite Substanz in Blut absorbiert wird, höher ist als eine Absorptionsrate, mit der der erste Laserstrahl bei der Zielmessung durch die erste Substanz in Blut absorbiert wird.

21. Verfahren für Messung einer Konzentration von Substanzen in Blut nach Anspruch 20, wobei die Konzentration der Referenzsubstanz in Blut stabiler ist als die Konzentration der Messungsziel-Substanz in Blut.

22. Verfahren für Messung einer Konzentration von Substanzen in Blut nach Anspruch 20, das des Weiteren umfasst:
Messen der Konzentration der zweiten Substanz in Blut in einem Zustand, in dem der bestimmte Bereich in dem Blutgefäßbereich (Mp) in dem Zielabschnitt (Mp0) enthalten ist, auf Basis von Emission des zweiten Laserstrahls, und
Messen der Konzentration der ersten Substanz in Blut in dem bestimmten Bereich als die Konzentration der ersten Substanz in Blut in dem Messungsziel-Abschnitt auf Basis von Emission des ersten Laserstrahls.

## Revendications

1. Dispositif de mesure de concentration d'une substance dans le sang (1 ; 1A) pour mesurer une concentration d'une substance dans le sang contenue dans le sang d'une partie objet (Mp0) d'un corps vivant (Ob), le dispositif de mesure de concentration d'une substance dans le sang (1 ; 1A) comprenant :
un socle (10) ayant une surface principale (10a) sur laquelle le corps vivant (Ob) peut être placé ;
une unité d'émission lumineuse (20) qui est disposée sur le côté surface principale (10a) du socle (10) et configurée pour condenser et émettre un faisceau laser (L1) vers une région spécifique dans la partie objet (Mp0), la région spécifique étant présente dans une peau située sur un côté du corps vivant (Ob), qui est le côté opposé par rapport à une surface cutanée sur un côté du corps vivant (Ob) faisant face à la surface principale (10a) ;
un photodétecteur (30) qui est disposé sur le côté surface principale (10a) du socle (10) et configuré pour recevoir une lumière de signal (L2) qui est une lumière réfléchie basée sur le faisceau laser (L1), et pour détecter une intensité de la lumière de signal (L2), la lumière de signal (L2) ayant, à certaines longueurs d'onde, une intensité lumineuse inférieure à celle du faisceau laser (L1) ;
une lentille d'imagerie (40) qui est disposée à une position entre la partie objet (Mp0) et le photodétecteur (30) où une image focalisée (Im2, Im21) de la lumière de signal (L2) provenant d'une région de condensation de faisceau laser (FA) dans la partie cible (Mp0) peut être formée sur le photodétecteur (30) ; et
un dispositif de commande de mesure (60 ; 60A) qui est configuré pour mesurer une concentration de la substance dans le sang dans la région de condensation de faisceau laser (FA) sur la base de l'intensité de la lumière de signal (L2), dans lequel la configuration est telle que :
un premier angle (θA) formé par une normale à la surface de la peau de la partie objet (Mp0) et un trajet optique (Op1) du faisceau laser (L1) est différent d'un second angle (θB) formé par la normale et un trajet optique (Op2) de la lumière de signal (L2) allant de la région de condensation de faisceau laser (FA) au photodétecteur (30),
la position du corps vivant (Ob) par rapport à l'unité d'émission lumineuse (20) est réglée de sorte que la région de condensation de faisceau laser (FA) et une région vasculaire (Mp) située dans l'intérieur de l'épiderme dans la partie objet (Mp0) se chevauchent ; et
la position du photodétecteur (30) par rapport au corps vivant (Ob) est réglée de sorte qu'une image (Im11) de la lumière de signal (L2) provenant de la région vasculaire (Mp) chevauchant la région de condensation de faisceau laser (FA) est transférée par la lentille d'imagerie (40) pour former une image focalisée (Im21) sur une surface de réception de lumière (30a) du photodétecteur (30).

2. Dispositif de mesure de concentration d'une substance dans le sang (1 ; 1A) selon la revendication 1, dans lequel
le socle (10) est capable d'ajuster la position du corps vivant (Ob) par rapport à l'unité d'émission lumineuse (20) en modifiant la hauteur (ht) de la surface principale (10a) dans une direction perpendiculaire à la surface principale (10a).

3. Dispositif de mesure de concentration d'une substance dans le sang (1 ; 1A) selon la revendication 1, dans lequel
la position du photodétecteur (30) par rapport au corps vivant (Ob) dans une direction entrecoupant le trajet optique (Op2) de la lumière de signal (L2) est capable d'être ajustée en modifiant la position du photodétecteur (30) dans ladite direction.

4. Dispositif de mesure de concentration d'une substance dans le sang (1 ; 1A) selon la revendication 1, dans lequel
le socle (10) est capable d'ajuster simultanément le premier angle (θA) et le second angle (θB) en modifiant l'angle (θC) de la surface principale (10a) par rapport au trajet optique (Op1) du faisceau laser (L1) dans un plan qui est défini par le trajet optique (Op1) du faisceau laser (L1) et le trajet optique (Op2) allant de la partie objet (Mp0) au photodétecteur (30).

5. Dispositif de mesure de concentration d'une substance dans le sang (1 ; 1A) selon la revendication 1, dans lequel
la position du photodétecteur (30) par rapport au corps vivant (Ob) dans une direction le long du trajet optique (Op2) de la lumière de signal (L2) est capable d'être ajustée en modifiant la position du photodétecteur (30) dans ladite direction, et
la position du photodétecteur (30) est ajustée sur la base de la concentration de la substance dans le sang de sorte que l'image (Im11) de la lumière de signal (L2) provenant de la région vasculaire (Mp) est transférée par la lentille d'imagerie (40) pour former une image focalisée (Im21) sur la surface de réception de lumière (30a) du photodétecteur (30).

6. Dispositif de mesure de concentration d'une substance dans le sang (1 ; 1A) selon l'une quelconque des revendications 1 à 5, dans lequel
l'unité d'émission lumineuse (20) est capable d'émettre sélectivement : un premier faisceau laser pour une mesure de cible qui est absorbé par une première substance dans le sang qui est une substance cible de mesure ; et un second faisceau laser pour une mesure de référence qui est absorbé par une seconde substance dans le sang qui est une substance de référence ; et
un taux d'absorption selon lequel le second faisceau laser est absorbé par la seconde substance dans le sang lors de la mesure de référence est supérieur à un taux d'absorption selon lequel le premier faisceau laser est absorbé par la première substance dans le sang lors de la mesure de cible.

7. Dispositif de mesure de concentration d'une substance dans le sang (1 ; 1A) selon la revendication 6, dans lequel
une concentration de la substance de référence dans le sang est plus stable qu'une concentration de la substance cible de mesure dans le sang.

8. Dispositif de mesure de concentration d'une substance dans le sang (1 ; 1A) selon la revendication 6, dans lequel
le dispositif de commande de mesure (60 ; 60A) est capable de mesurer, sur la base de l'émission du second faisceau laser, une concentration de la seconde substance dans le sang dans un état où la région spécifique est contenue dans la région vasculaire (Mp) de la partie objet (Mp0), et
est capable de mesurer, sur la base de l'émission du premier faisceau laser, une concentration de la première substance dans le sang dans la région spécifique en tant que concentration de la première substance dans le sang dans la partie cible de mesure.

9. Dispositif de mesure de concentration d'une substance dans le sang (1 ; 1A) selon la revendication 1, comprenant en outre
une lentille condensatrice (50) qui est positionnée entre l'unité d'émission lumineuse (20) et la partie objet (Mp0) dans le trajet optique (Op1) du faisceau laser (L1), et qui condense le faisceau laser (L1) vers la région de condensation de faisceau laser (FA).

10. Dispositif de mesure de concentration d'une substance dans le sang (1 ; 1A) selon la revendication 9, dans lequel
dans une section allant du socle (10) au photodétecteur (30) dans le trajet optique (Op2) allant de la partie objet (Mp0) au photodétecteur (30), la lumière de signal (L2) se propage à travers un espace, à l'exception d'une section dans laquelle la lumière de signal (L2) traverse la lentille d'imagerie (40) ; et
dans une section allant de l'unité d'émission lumineuse (20) au socle (10) dans le trajet optique (Op1) allant de l'unité d'émission lumineuse (20) à la partie objet (Mp0), le faisceau laser (L1) se propage à travers un espace, à l'exception d'une section dans laquelle le faisceau laser (L1) traverse la lentille condensatrice (50).

11. Dispositif de mesure de concentration d'une substance dans le sang (1 ; 1A) selon l'une quelconque des revendications 1 à 5 ou la revendication 9, comprenant en outre des moyens de capture d'image (91A ; 92A) qui sont configurés pour capturer une image comportant le corps vivant (Ob), dans lequel
le dispositif de commande de mesure (60 ; 60A) est configuré pour détecter, à partir de l'image acquise, une portion d'image correspondant au corps vivant (Ob) et pour calculer une quantité d'écart de position par rapport à une position de référence où la portion d'image devrait se trouver ; et
le socle (10) est capable de modifier la hauteur (ht) de la surface principale (10a) dans une direction perpendiculaire de manière à compenser la quantité d'écart de position.

12. Dispositif de mesure de concentration d'une substance dans le sang (1 ; 1A) selon l'une quelconque des revendications 1 à 5 ou la revendication 9, comprenant en outre des moyens de capture d'image (91A ; 92A) qui sont configurés pour capturer une image comportant le corps vivant (Ob), dans lequel
le dispositif de commande de mesure (60 ; 60A) est configuré pour détecter, à partir de l'image acquise, une portion d'image correspondant au corps vivant (Ob) et pour calculer une quantité d'écart angulaire par rapport à un angle de référence selon lequel ladite portion d'image devrait être orientée ; et
le socle (10) est capable de modifier l'angle (θC) de la surface principale (10a) par rapport au trajet optique (Op1) du faisceau laser (L1) de manière à compenser la quantité d'écart angulaire.

13. Dispositif de mesure de concentration d'une substance dans le sang (1 ; 1A) selon la revendication 1, comprenant en outre
un mécanisme d'ajustement d'angle d'émission lumineuse (93B) qui maintient l'unité d'émission lumineuse (20) et le photodétecteur (30) et qui est configuré, par rotation, pour modifier l'angle (θB) du trajet optique (Op2) de la lumière de signal (L2) et l'angle (θA) du trajet optique (Op1) du faisceau laser (L1) par rapport à la surface de la peau du corps vivant (Ob) dans un plan d'incidence du faisceau laser.

14. Dispositif de mesure de concentration d'une substance dans le sang (1 ; 1A) selon la revendication 13, comprenant en outre
un mécanisme d'ajustement de position (12A ; 94B) qui est configuré pour modifier la position du mécanisme d'ajustement d'angle d'émission lumineuse (93B) dans le plan d'incidence du faisceau laser.

15. Procédé de mesure de concentration d'une substance dans le sang pour mesurer une concentration d'une substance dans le sang contenue dans le sang d'une partie objet (Mp0) d'un corps vivant (Ob), le procédé de mesure de concentration d'une substance dans le sang comprenant les étapes consistant à :
placer le corps vivant (Ob) sur une surface principale (10a) d'un socle (10), et
condenser et émettre un faisceau laser (L1) pour une mesure de cible vers une région spécifique dans la partie objet (Mp0) au moyen d'une unité d'émission lumineuse (20) qui est disposée sur le côté surface principale (10a) du socle (10), la région spécifique étant présente dans une peau située sur un côté du corps vivant (Ob) qui est le côté opposé par rapport à une surface cutanée sur un côté du corps vivant (Ob) faisant face à la surface principale (10a), et le faisceau laser (L1) pour la mesure de cible étant absorbé par une substance dans le sang qui est une substance cible de mesure ;
utiliser une lentille d'imagerie (40) qui est positionnée entre la partie objet (Mp0) et un photodétecteur (30), qui est disposé sur le côté surface principale (10a) du socle (10),
pour former une image focalisée (Im2, Im21) de lumière de signal (L2) sur le photodétecteur (30), la lumière de signal (L2) étant une lumière réfléchie du faisceau laser (L1), étant émise depuis une région de condensation de faisceau laser (FA) dans la partie objet (Mp0), et ayant, à certaines longueurs d'onde, une intensité lumineuse inférieure à celle du faisceau laser (L1) ; et
recevoir la lumière de signal (L2) au moyen du photodétecteur (30) et mesurer une concentration de la substance dans le sang dans la région spécifique sur la base de l'intensité de la lumière de signal (L2) reçue, dans lequel
un premier angle (θA) formé par une normale à la surface de la peau de la partie objet (Mp0) et un trajet optique (Op1) du faisceau laser (L1) est différent d'un second angle (θB) formé par la normale et un trajet optique (Op2) de la lumière de signal (L2) allant de la région spécifique au photodétecteur (30),
la position du corps vivant (Ob) par rapport à l'unité d'émission lumineuse (20) est réglée de sorte que la région de condensation de faisceau laser (FA) et une région vasculaire (Mp) située dans l'intérieur de l'épiderme dans la partie objet (Mp0) se chevauchent ; et
la position du photodétecteur (30) par rapport au corps vivant (Ob) est réglée de sorte qu'une image (Im11) de la lumière de signal (L2) provenant de la région vasculaire (Mp) chevauchant la région de condensation de faisceau laser (FA) est transférée par la lentille d'imagerie (40) pour former une image focalisée (Im21) sur une surface de réception de lumière (30a) du photodétecteur (30).

16. Procédé de mesure de concentration d'une substance dans le sang selon la revendication 15, comprenant en outre
avant la mesure, l'ajustement de la position du corps vivant (Ob) par rapport à l'unité d'émission lumineuse (20) en modifiant la hauteur (ht) de la surface principale (10a) du socle (10) dans une direction perpendiculaire à la surface principale (10a).

17. Procédé de mesure de concentration d'une substance dans le sang selon la revendication 15, comprenant en outre
avant la mesure, l'ajustement de la position du photodétecteur (30) par rapport au corps vivant (Ob) dans une direction entrecoupant le trajet optique (Op2) de la lumière de signal (L2) en modifiant la position du photodétecteur (30) dans ladite direction.

18. Procédé de mesure de concentration d'une substance dans le sang selon la revendication 15, comprenant en outre
avant la mesure de cible, l'ajustement simultané du premier angle (θA) et du second angle (θB) sur la base de la concentration de la substance dans le sang en réalisant une mesure de référence dans laquelle la concentration de la substance dans le sang dans la région spécifique est mesurée tout en modifiant l'angle (θC) de la surface principale (10a) dans un plan qui est défini par le trajet optique (Op1) du faisceau laser (L1) et le trajet optique (Op2) allant de la partie objet (Mp0) au photodétecteur (30).

19. Procédé de mesure de concentration d'une substance dans le sang selon la revendication 15, comprenant en outre
avant la mesure de cible, l'ajustement de la position du photodétecteur (30) par rapport au corps vivant (Ob) dans une direction le long du trajet optique (Op2) de la lumière de signal (L2) en réalisant une mesure de référence dans laquelle la concentration de la substance dans le sang est mesurée tout en modifiant la position du photodétecteur (30) le long du trajet optique (Op2) de la lumière de signal (L2), dans lequel lors de l'ajustement, la position du photodétecteur (30) est ajustée sur la base de la concentration de la substance dans le sang de sorte qu'une image (Im11) de la lumière de signal (L2) provenant de la région vasculaire (Mp) est transférée par la lentille d'imagerie (40) pour former une image focalisée (Im21) sur la surface de réception de lumière (30a) du photodétecteur (30).

20. Procédé de mesure de concentration d'une substance dans le sang selon l'une quelconque des revendications 15 à 19, dans lequel
lorsque le faisceau laser, la substance dans le sang et la concentration de la substance dans le sang sont respectivement désignés un premier faisceau laser, une première substance dans le sang, et une concentration de la première substance dans le sang, lors de la mesure de référence :
un second faisceau laser pour la mesure de référence, qui est absorbé par une seconde substance dans le sang qui est une substance de référence, est émis par l'unité d'émission lumineuse (20) vers la région irradiée ;
à l'aide de la lentille d'imagerie (40), une image focalisée (Im21) est formée sur le photodétecteur (30) à partir de la lumière de signal (L2) du second faisceau laser réfléchi par la région spécifique ;
la lumière de signal (L2) du second faisceau laser est reçue au moyen du photodétecteur (30), et une concentration de la seconde substance dans le sang basée sur la lumière de signal (L2) est mesurée en tant que concentration de la seconde substance dans le sang dans la partie cible de mesure ; et
un taux d'absorption selon lequel le second faisceau laser est absorbé par la seconde substance dans le sang est supérieur à un taux d'absorption selon lequel le premier faisceau laser est absorbé par la première substance dans le sang lors de la mesure de cible.

21. Procédé de mesure de concentration d'une substance dans le sang selon la revendication 20,
dans lequel la concentration de la substance de référence dans le sang est plus stable que la concentration de la substance cible de mesure dans le sang.

22. Procédé de mesure de concentration d'une substance dans le sang selon la revendication 20, comprenant en outre :
la mesure, sur la base de l'émission du second faisceau laser, de la concentration de la seconde substance dans le sang dans un état où la région spécifique est contenue dans la région vasculaire (Mp) de la partie objet (Mp0) ; et
la mesure, sur la base de l'émission du premier faisceau laser, de la concentration de la première substance dans le sang dans la région spécifique en tant que concentration de la première substance dans le sang dans la partie cible de mesure.
